# EUROPEAN PATENT APPLICATION

(11) **EP 4 613 759 A1**
(43) Date of publication of application: **10.09.2025**
(21) Application number: 23884931.9
(22) Date of filing: 31.10.2023
(51) Int. Cl.: C07K 14/00, C07K 19/00, C12N 15/62, C12N 15/70, C12N 1/21, G01N 33/533, G01N 33/58, G01N 33/53, G01N 21/00, C12R 1/19

(54) **FRUCTOSE-1,6-DIPHOSPHATE OPTICAL PROBE, AND PREPARATION METHOD THEREFOR AND USE THEREOF**

(30) Priority: 31.10.2022 CN 202211345446
(71) Applicant: East China University of Science and Technology, Shanghai 200237 (CN)
(72) Inventor: YANG, Yi, Shanghai 200237 (CN); ZHAO, Yuzheng, Shanghai 200237 (CN); YAO, Jing, Shanghai 200237 (CN); ZOU, Yejun, Shanghai 200237 (CN); ZHANG, Lijuan, Shanghai 200237 (CN); CHEN, Yan, Shanghai 200237 (CN); LI, Xie, Shanghai 200237 (CN)
(74) Representative: Nederlandsch Octrooibureau
(86) International application number: PCT/CN2023/128488
(87) International publication number: WO 2024/094002

(57) **Abstract**

The present invention relates to a fructose diphosphate optical probe. Specifically, provided in the present invention is a fructose diphosphate optical probe, which comprises a fructose-1,6-diphosphate sensitive polypeptide and an optically active polypeptide, wherein the optically active polypeptide is located within the sequence of a fructose-1,6-diphosphate sensitive polypeptide, the fructose-1,6-diphosphate sensitive polypeptide is a fructose-1,6-diphosphate binding protein or a functional variant thereof, and the optically active polypeptide is a fluorescent protein or a functional variant thereof. The fluorescent probe of the present invention has a large dynamic change in terms of fluorescence and a good specificity, and can be used for high-throughput and quantitative detection of fructose diphosphate inside and outside a cell.

## Description

### Field of the disclosure

The present invention relates to the field of optical probe technology, especially to a fructose-1,6-diphosphate optical probe, preparation method thereof and application thereof.

### Background of the disclosure

Fructose-1,6-bisphosphate (FBP) is a naturally occurring compound in cells and an important intermediate product produced by cells in the glycolysis pathway. It produces pharmacological effects in cells by regulating the activities of several enzymes in sugar metabolism.

Fructose-1,6-bisphosphate is an important metabolite in the glycolysis pathway and gluconeogenesis process. It is produced by fructose-6-phosphate under the catalysis of phosphofructokinase, and then generates dihydroxyacetone phosphate and 3-phosphoglyceraldehyde under the action of aldolase, and finally generates pyruvate and enters the tricarboxylic acid cycle pathway. In addition, fructose-1,6-bisphosphate regulates the activity of multiple enzymes in multiple metabolic pathways.

Due to its important role in metabolic pathways, fructose-1,6-bisphosphate is used to treat metabolic-related and respiratory-related diseases, and has achieved good therapeutic effects. For example, when sugar metabolism in the body is abnormal, the ATP content decreases, which in turn leads to a decrease in the oxygen-carrying capacity of hemoglobin, causing metabolic disorders and diseases. Fructose-1,6-bisphosphate can promote the decomposition of sugar in cells and treat related diseases. In terms of respiratory diseases, fructose-1,6-bisphosphate increases the content of the phosphate pool in the body by promoting the decomposition of intracellular phosphofructokinase, thereby increasing the relaxation capacity of lung smooth muscles and repairing damaged cells.

Currently, the common methods for detecting fructose-1,6-bisphosphate mainly include chromatography (Ma B et al. European Journal of Pharmacology, 2013, 718 (1-3), 524-532), enzymatic method (Iwamoto S et al. Applied and Environmental Microbiology, 2007, 73 (17), 5676-5678), and colorimetric method (Wang C et al. Journal of biomedical engineering, 2000, 17 (3), 363-365). Chromatographic and enzymatic detection have good specificity, but have the disadvantage of high cost (Wu Liangyong et al. Chinese Pharmaceutical Standards, 2007, 8 (4), 63-65);chromatographic method can quickly detect the content of fructose-1,6-diphosphate, and can detect low content of fructose-1,6-diphosphate, but errors are prone to occur in the experimental process (Wu Yan et al. Journal of Central and Southern Medical Sciences, 2000, (6), 608-609); and the colorimetric method has the problem of interference from substances such as fructose and fructose monophosphate (Du Zhenning et al. Chinese Journal of Biochemical Pharmacy, 1993, (2), 59-62), and the above methods are only suitable for in vitro detection, and cannot monitor the concentration changes of fructose-1,6-diphosphate in living cells in real time. Therefore, it is of great significance to develop a new method with good specificity, fast response speed and real-time quantitative detection of fructose-1,6-diphosphate in vivo.

### Summary of the disclosure

The purpose of the present invention is to provide probes and methods for real-time localization, high-throughput, and quantitative detection of fructose-1,6-diphosphate in and out of cells.

To achieve the above object, the invention provides the following technical solutions:
The first aspect of the present invention provides a fructose-1,6-bisphosphate binding protein variant, which:
(1) having a sequence as shown in SEQ ID NO: 1 and having a mutation at one, two, three, four, five or more positions selected from the group consisting of:T148, R175, G176, G177, L178, E180, D181, V182, K183, N184, Q185, F271, and wherein the mutation comprises a modification, substitution or deletion of an amino acid,
(2) is a truncated variant of (1) having amino acids 88 to 340, or
(3) is a sequence having at least 70% sequence identity with the sequence of (1) or (2) and having the mutation described in (1) and retaining the ability to bind to fructose-1,6-bisphosphate.

In one or more embodiments, the mutated positions are selected from 1, 2 or 3, 4, or 5 of any one of the following groups: (a) T148, R175, G176, G177, L178, D181, V182, K183, N184, Q185, (b) G177, L178, E180, D181, V182, N184, Q185, F271.

In one or more embodiments, the mutation comprises mutations at positions selected from any one of the following groups: (1) G177 and L178, (5) D181 and V182, (6) E180 and D181, (7) N184 and Q185; or, the mutation comprises a mutation at a position selected from any one of the following groups: (1) G177, L178, (2) G177, L178 and Q185, (3 )G177, L178 and T148, (4) G177, L178 and F271, (5) D181, V182, (6) E180, D181, (7) N184, Q185, (8) N184, Q185, D181, V182, G177, (9) N184, Q185, G177, (10) N184, Q185, D181, V182.

In one or more embodiments, T148 is mutated to S. In one or more embodiments, G177 is mutated to A, I, L, M or N, preferably to L or M. In one or more embodiments, L178 is mutated to Y, F or N, preferably to N. In one or more embodiments, E180 is mutated to G. In one or more embodiments, D181 is mutated to F, L or E, preferably to E. In one or more embodiments, V182 is mutated to F, L or G, preferably to G. In one or more embodiments, N184 is mutated to V. In one or more embodiments, Q185 is mutated to S, A, T, D or P. In one or more embodiments, F271 is mutated to Y.

In one or more embodiments, the mutation comprises one or more selected from the group consisting of: G177L, D181E, V182G, N184V, Q185A.

In one or more embodiments, the mutation comprises a mutation selected from any one of the following groups: (1) G177A and L178Y, (2) G1771 and L178F, (3) G177L and L178Y, (4) G177M and L178N, (5) G177N and L178Y, (6) G177M, L178N and Q185S, (7) G177M, L178N and Q185A, (8) G177M, L178N and Q185T, (9) G177M, L178N and Q185D, (10) G177M, L178N and T148S, (11) G177M, L178N and F271Y, (12) D181F and V182F, (13) D181L and V182L, (14) E180G and D181G, (15) N184V and Q185P, (16) N184V, Q185P, D181E, V182G and G177L, (17) N184V, Q185P and G177L, (18) N184V, Q185P, D181E and V182G.

Another aspect of the present invention provides a fructose-1,6-bisphosphate optical probe, comprising a fructose-1,6-bisphosphate sensitive polypeptide and an optically active polypeptide, wherein the optically active polypeptide is located within the sequence of the fructose-1,6-bisphosphate sensitive polypeptide. The fructose-1,6-bisphosphate-sensitive polypeptide is divided into a first part and a second part by the optically active polypeptide.

In one or more embodiments, the fructose-1,6-bisphosphate optical probe comprising a fructose-1,6-bisphosphate sensitive polypeptide B and optically active polypeptide A, wherein the optically active polypeptide A is located in the sequence of the fructose-1,6-bisphosphate sensitive polypeptide B, dividing the fructose-1,6-bisphosphate sensitive polypeptide B into the first part B1 and the second part B2, forming a probe structure of type B1-A-B2.

In one or more embodiments, the optically active polypeptide is located between residues 174-185 and/or 201-208 of the fructose-1,6-bisphosphate sensitive polypeptide, numbered corresponding to the full length of the fructose-1,6-bisphosphate sensitive polypeptide. Preferably, the optically active polypeptide is located at any one or more of the following positions of the fructose-1,6-bisphosphate sensitive polypeptide: 174/175, 174/176, 174/177, 174/178, 174/179, 174/180, 174/181, 174/182, 174/183, 174/184, 174/185, 175/176, 175/177, 175/178, 175/179, 175/180, 175/181, 175/182, 175/183, 175/184, 175/185 75/185, 176/177, 176/178, 176/179, 176/180, 176/181, 176/182, 176/183, 176/184, 176/185, 177/178, 177/179, 177/180, 177/181, 177/182, 177/183, 177/184, 177/185, 178/179, 178/180, 178/181, 178/182, 178/183, 178/184, 178/185, 179/ 180, 179/181, 179/182, 179/183, 179/184, 179/185, 180/181, 180/182, 180/183, 180/184, 180/185, 181/182, 181/182, 181/183, 181/184, 181/185, 182/183, 182/184, 182/185, 183/184, 183/185, 184/185, 201/202, 201/203, 201/204, 201/205 , 201/206, 201/207, 201/208, 202/203, 202/204, 202/205, 202/206, 202/207, 202/208, 203/204, 203/205, 203/206, 203/207, 203/208, 204/205, 204/206, 204/207, 204/208, 205/206, 205/206, 205/207, 205/208, 206/207, 206/208 and/or 207/208. More preferably, the optically active polypeptide is located at any one or more of the following positions of the fructose-1,6-bisphosphate-sensitive polypeptide: 176/177, 176/178, 177/181, 179/180, 182/184, 182/185, 203/206, 203/207 and 203/208.

In one or more embodiments, the fructose-1,6-bisphosphate-sensitive polypeptide is a fructose-1,6-bisphosphate binding protein or a functional variant thereof, wherein the functional variant of the fructose-1,6-bisphosphate binding protein has a mutation within 7 amino acids at the position where it is connected to the optically active polypeptide.

In one or more embodiments, the fructose-1,6-bisphosphate-sensitive polypeptide has:
(1) the sequence shown in SEQ ID NO: 1 or a truncated variant thereof having amino acids 88 to 340, or a sequence having at least 70% sequence identity therewith and retaining fructose-1,6-bisphosphate binding activity,
(2) the sequence of the fructose-1,6-bisphosphate binding protein variant described in any embodiment of the first aspect of the present invention, or
(3) a sequence having at least 70% sequence identity with the sequence described in (2) and having the mutation described in (2) and retaining sensitivity to fructose-1,6-bisphosphate.

In one or more embodiments, the optically active polypeptide is a fluorescent protein or a functional variant thereof, wherein the functional variant of the fluorescent protein has a mutation within 3 amino acids at the junction with the optically active polypeptide.

In one embodiment, the fluorescent protein is selected from yellow fluorescent protein, orange fluorescent protein, red fluorescent protein, green fluorescent protein, blue fluorescent protein, apple red fluorescent protein. In one embodiment, the fluorescent protein has a sequence as shown in any of SEQ ID NO: 2-9.

In one or more embodiments, the functional variant of the fluorescent protein has a mutation at amino acids 1-3, preferably at position 1. Preferably, the functional variant of the fluorescent protein comprises a mutation in which the first amino acid of the fluorescent protein is mutated to I or V.

In one or more embodiments, the functional variant of the fluorescent protein has the sequence shown in SEQ ID NO: 2 and has a mutation at the Y1 position. Preferably, the mutation is Y1I or Y1V.

In one embodiment, the optical probe further comprises one or more linkers flanking the optically active polypeptide. The linker of the present invention can be any amino acid sequence of any length. In one embodiment, the optically active polypeptide is flanked by a linker of no more than 5 amino acids, such as a linker of 0, 1, 2, 3, 4 amino acids. In one embodiment, the linker flanking the optically active polypeptide comprises amino acid Y. In one embodiment, linker Y is located N-terminal and/or C-terminal to the optically active polypeptide. In one embodiment, the optical probe is shown as follows: the first part B1 of the of the fructose-1,6-bisphosphate sensitive polypeptide, Y, optically active polypeptide A, the second part B2 of the fructose-1,6-bisphosphate sensitive polypeptide. In one embodiment, the present optical probe does not comprise a linker.

In one embodiment, the optical probe of the present invention further comprises a localization sequence for localizing the probe to, for example, a specific organelle of a cell.

In one or more embodiments, the fructose-1,6-bisphosphate sensitive polypeptide is a truncated variant of SEQ ID NO: 1 having amino acids 88-340, and the optically active polypeptide is located at any one or more of the following positions of the fructose-1,6-bisphosphate sensitive polypeptide: 176/177, 176/178, 177/181, 179/180, 182/184, 182/185, 203/206, 203/207 and 203/208. Preferably, the optically active polypeptide is as shown in SEQ ID NO: 2.

In one or more embodiments, the fructose-1,6-bisphosphate sensitive polypeptide is a truncated variant of SEQ ID NO: 1 having amino acids 88-340, the optically active polypeptide is located at any one or more of the following positions of the fructose-1,6-bisphosphate sensitive polypeptide: 176/177, 177/181, 179/180 or 182/184, and the optical probe has one or more mutations selected from the group consisting of: T148S, G177A, G177I, G177L, G177 M, G177N, L178Y, L178F, L178Y, L178N, L178Y, L178N, E180G, D181E, D181F, D181L, D181G, V182F, V182L, V182G, N184V, Q185A, Q185S, Q185A, Q185T, Q185D, Q185P, F271Y, the first amino acid of the optically active polypeptide is mutated to V or I. Preferably, the optically active polypeptide is as shown in SEQ ID NO: 2.

In one or more embodiments, the fructose-1,6-bisphosphate sensitive polypeptide is a truncated variant of SEQ ID NO: 1 having amino acids 88-340, the optically active polypeptide is located at any one or more of the following positions of the fructose-1,6-bisphosphate-sensitive polypeptide: 176/177, 177/181, 179/180 or 182/184, and the optical probe has a mutation selected from any one of the following groups: (1) G177A and L178Y of the fructose-1,6-bisphosphate sensitive polypeptide, (2) G177I and L178F of the fructose-1,6-bisphosphate sensitive polypeptide, (3) G177L and L178Y of the fructose-1,6-bisphosphate sensitive polypeptide, (4) G177M and L178N of the fructose-1,6-bisphosphate sensitive polypeptide, (5) G177N and L178Y of fructose-1,6-bisphosphate sensitive polypeptide, (6) G177M, L178N and Q185S of fructose-1,6-bisphosphate sensitive polypeptide, (7) G177M, L178N and Q185A of fructose-1,6-bisphosphate sensitive polypeptide, (8) G177M, L178N and Q185T of fructose-1,6-bisphosphate sensitive polypeptide, (9) G177M, L178N and Q185D of fructose-1,6-bisphosphate sensitive polypeptide, (10) G177M, L178N and T148S of fructose-1,6-bisphosphate sensitive polypeptide, (11)G177M, L178N and F271Y of a phosphate sensitive polypeptide, (12) D181F and V182F of a fructose-1,6-bisphosphate sensitive polypeptide, (13) D181L and V182L of a fructose-1,6-bisphosphate sensitive polypeptide, (14) E180G and D181G of a fructose-1,6-bisphosphate sensitive polypeptide, (15) N184V and Q185P of a fructose-1,6-bisphosphate sensitive polypeptide, (16) N184V, Q185P, D181E, V182G, G177L of a fructose-1,6-bisphosphate sensitive polypeptide, and the first amino acid of an optically active polypeptide is mutated to V, (17) N184V, Q185P, D181E, V182G, G177L of the phosphate-sensitive polypeptide, and the first amino acid of the optically active polypeptide are mutated to I, (18) N184V, Q185P and G177L of the fructose-1,6-bisphosphate-sensitive polypeptide, and the first amino acid of the optically active polypeptide are mutated to V, (19) N184V, Q185P and G177L of the fructose-1,6-bisphosphate-sensitive polypeptide, and the first amino acid of the optically active polypeptide are mutated to I, (20) N184V, Q185P, D181E and V182G of the fructose-1,6-bisphosphate-sensitive polypeptide, and the amino acid at position 1 of the optically active polypeptide are mutated to V. Preferably, the optically active polypeptide is as shown in SEQ ID NO: 2.

Another aspect of the invention also provides fusion polypeptides comprising the optical probes described herein and other polypeptides. In some embodiments, other polypeptides are located N- and/or C-terminal to the said optical probe. In some embodiments, other polypeptides include polypeptides that localize optical probes to different organelles or subcellular organelles, tags for purification, or tags for immunoblotting.

Another aspect of the present invention provides a nucleic acid molecule comprising: (a) a coding sequence of a polypeptide or probe as described in any embodiment of the present invention, or (b) a complementary sequence of (a), or (c) a fragment of (a) or (b). The fragment is primer.

The invention also relates to variants of the nucleic acid sequences described above, including nucleic acid sequences encoding the fragments, analogs, derivatives, soluble fragments and variants of the optical probes or fusion proteins of the invention or the complementary sequences thereof.

Another aspect of the present invention provides a nucleic acid construct comprising the nucleic acid molecule in the present invention. The nucleic acid sequence encodes the optical probe or fusion polypeptide described in the present invention.

In one or more embodiments, the nucleic acid construct is a cloning vector, expression vector, or recombinant vector.

In one or more embodiments, the nucleic acid molecule is operably linked to an expression control sequence.

In some embodiments, the expression vector is selected from the group consisting of prokaryotic expression vector, eukaryotic expression vector, and viral vector.

Another aspect of the present invention provides a host cell, wherein the host cell: (1) expresses the optical probe or fusion polypeptide according to any embodiment of the present invention; (2) comprises the nucleic acid molecule according to any embodiment of the present invention; or (3) comprises the nucleic acid construct according to any embodiment of the present invention. The host cell is preferably Escherichia coli.

Another aspect of the present invention provides a fructose-1,6-bisphosphate detection kit, comprising the optical probe or fusion polypeptide or polynucleotide described herein or the optical probe prepared by the method described herein.

In one or more embodiments, the kit further comprises one or more reagents selected from the group consisting of a buffer, a culture medium, and a fructose-1,6-bisphosphate standard.

Another aspect of the present invention provides a method for preparing the optical probes, comprising providing host cells expressing the optical probes or fusion polypeptides described herein, culturing the host cells under conditions in which the cells express them, and isolating the optical probes or fusion polypeptides.

In one or more embodiments, the method includes the following steps: 1) incorporating the nucleic acid sequence encoding the the fructose-1,6-bisphosphate optical probe described herein into an expression vector; 2) transferring the expression vector into the host cell; 2) culturing the host cell under conditions suitable for the expression of the expression vector, 3) separation of the fructose-1,6-bisphosphate optical probe.

Another aspect of the present invention provides a method for detecting fructose-1,6-bisphosphate in a sample, comprising: contacting the optical probe or fusion polypeptide or host cell described herein with the sample, and detecting changes in the optically active polypeptide. The detecting may be performed in vivo, in vitro, subcellularly, or in situ. The sample is such as blood.

Another aspect of the present invention provides a method for quantifying fructose-1,6-bisphosphate in a sample, comprising: contacting the optical probe or fusion polypeptide or host cell described in the present invention with the sample, detecting the optical change of the optically active polypeptide, and quantifying the fructose-1,6-bisphosphate in the sample based on the optical change of the optically active polypeptide.

Another aspect of the present invention provides a method for screening compounds (e.g., drugs), comprising: contacting the optical probe or fusion polypeptide described herein or a host cell with a candidate compound in a system containing fructose-1,6-bisphosphate, detecting the optical change of the optically active polypeptide, and screening the compound based on the optical change of the optically active polypeptide. The method allows high-throughput screening of compounds.

In one or more embodiments, a host cell described herein is contacted with a candidate compound in a system containing fructose-1,6-bisphosphate, and an optical change in the optically active polypeptide indicates whether the candidate compound can modulate the uptake of fructose-1,6-bisphosphate by the cell.

Another aspect of the present invention provides a method for localizing the fructose-1,6-bisphosphate in and/or out of the cell, comprising: contacting a system containing fructose-1,6-bisphosphate with the optical probe or the host cell, and detecting optical changes of the optically active polypeptide.

In one or more embodiments, the system is a solution system, a cellular system, or a subcellular system.

Another aspect of the present invention provides the use of the fructose-1,6-bisphosphate optical probe or fusion polypeptide or host cell described herein in detecting fructose-1,6-bisphosphate in a sample, screening compounds or fructose-1,6-bisphosphate intracellular/extracellular localization. In one or more embodiments, the localization is in real time.

**Beneficial effects of the present invention:** The fructose-1,6-bisphosphate optical probe provided by the invention is easy to mature, has large dynamic change of fluorescence and good specificity and it can be expressed in cells through the method of gene manipulation, and it can locate, high-throughput, and quantitatively detect fructose-1,6-bisphosphate in and out of cells in real time, eliminating the time-consuming steps of sample processing. The experimental results show that the highest response of the fructose-1,6-bisphosphate optical probe provided in this application to fructose-1,6-bisphosphate is more than 12 times that of the control, and it can locate, qualitatively and quantitatively detect cells in subcellular structures such as cytoplasm, mitochondria, nucleus, endoplasmic reticulum, lysosome and Golgi apparatus, and can perform high-throughput compound screening and quantitative detection of fructose-1,6-bisphosphate in blood.

### Description of drawings

The invention is further illustrated below in combination with the drawings and examples.
Figure 1 is an SDS-PAGE graph of the exemplary fructose-1,6-bisphosphate optical probe described in Example 2;
Figure 2 is a table showing the response changes of the exemplary fructose-1,6-bisphosphate optical probe comprising cpYFP and fructose-1,6-bisphosphate binding protein to fructose-1,6-bisphosphate described in Example 2;
Figure 3 is a table showing the response changes of the exemplary fructose-1,6-bisphosphate optical probe comprising cpGFP and fructose-1,6-bisphosphate binding protein to fructose-1,6-bisphosphate described in Example 3 ;
Figure 4 is a table showing the response changes of the exemplary fructose-1,6-bisphosphate optical probe comprising cpBFP and fructose-1,6-bisphosphate binding protein to fructose-1,6-bisphosphate described in Example 4;
Figure 5 is a fluorescence spectrum properties graph of the exemplary fructose-1,6-bisphosphate optical probe described in Example 6;
Figure 6 is the titration curve of the exemplary fructose-1,6-bisphosphate optical probe to different concentrations of fructose-1,6-bisphosphate described in Example 6;
Figure 7 is a bar graph showing the specific detection of multiple similar substrates in the glycolytic pathway by the exemplary fructose-1,6-bisphosphate optical probe described in Example 6;
Figure 8 is a the subcellular organelle localization photograph of the exemplary fructose-1,6-bisphosphate optical probe in mammalian cells described in Example 7;
Figure 9 is a schematic diagram of the dynamic monitoring of cytoplasmic fructose-1,6-bisphosphate concentration in the cytoplasm of mammalian cells with an exemplary fructose-1,6-bisphosphate optical probe described in Example 7;
Figure 10 is a dot plot of the exemplary fructose-1,6-bisphosphate optical probe for high-throughput compound screening at the live cell level described in Example 8;
Figure 11 is a bar graph showing the quantitative determination of fructose-1,6-bisphosphate in human blood by the exemplary fructose-1,6-bisphosphate optical probe described in Example 9.

### Detailed description

When a value or range is given, the term "about" used herein means that the value or range is within 20%, within 10%, and within 5% of the given value or range.

The terms "comprise", "include", and equivalent forms thereof include the meaning of "contain" as well as "consist of", for example a composition "comprising" X may consist of X alone or may contain other substances, such as X + Y.

The term "fructose-1,6-bisphosphate-sensitive polypeptide" used herein refers to a polypeptide which responds to fructose-1,6-bisphosphate including any response in chemical, biological, electrical, or physiological parameters of a polypeptide that interacts with the sensitive-polypeptide. The response includes small changes, for example, changes in the orientation of the amino acid or peptide fragment of the polypeptide and, for example, changes in the primary, secondary or tertiary structure of the polypeptide, including, for example, changes in protonation, electrochemical potential and/or conformation. "Conformation" is the three-dimensional arrangement of the primary, secondary, and tertiary structure of a molecule containing side groups in the molecule; when the three-dimensional structure of the molecule changes, the conformation changes. Examples of conformational changes include transitions from α-helix to β-fold or from β-fold to α-helix. It should be understood that detectable alterations need not be conformational changes as long as the fluorescence of the fluorescent protein moiety is altered. The fructose-1,6-bisphosphate sensitive polypeptide described herein may also include functional variants thereof. The functional variants of the fructose-1,6-bisphosphate sensitive polypeptide include, but are not limited to, variants that can interact with fructose-1,6-bisphosphate and thereby undergo the same or similar changes as the parent fructose-1,6-bisphosphate sensitive polypeptide.

The fructose-1,6-bisphosphate sensitive polypeptides of the present invention include, but are not limited to, the fructose-1,6-bisphosphate binding protein CggR or its variants with more than 90% homology. The exemplary fructose-1,6-bisphosphate binding protein CggR of the present invention is derived from *Bacillus subtilis.* CggR belongs to the SorC/DeoR family of prokaryotic transcriptional regulatory factors and consists of two domains: the N-terminal DNA binding domain and the C-terminal ligand domain. Fructose-1,6-bisphosphate binding protein can sense changes in fructose-1,6-bisphosphate concentration, and the spatial conformation of fructose-1,6-bisphosphate binding protein will also change during the dynamic change of fructose-1,6-bisphosphate concentration. An exemplary CggR protein is shown in SEQ ID NO:1. When describing the optical probes of the invention (eg, when describing insertion positions or mutation positions), references to amino acid residue numbering refer to SEQ ID NO:1.

The term "optical probe" as used herein refers to a fructose-1,6-bisphosphate sensitive polypeptide fused to an optically active polypeptide. The inventors discovered that the conformational changes produced by binding a fructose-1,6-bisphosphate sensitive polypeptide, such as a fructose-1,6-bisphosphate binding protein to a physiological concentration of fructose-1,6-bisphosphate specifically by fructose-1,6-bisphosphate sensitive polypeptides causes conformational changes in an optically active polypeptides, such as fluorescent proteins, which in turn result in the alteration of the optical properties of the optically active polypeptides. A standard curve is plotted with the help of the fluorescence of fluorescent proteins determined at different concentrations of fructose-1,6-bisphosphate allows the presence and/or level of fructose-1,6-bisphosphate to be detected and analyzed.

In the optical probe of the present invention, an optically active polypeptide, such as a fluorescent protein, is operably inserted into a fructose-1,6-bisphosphate sensitive polypeptide. The protein-based "optically active polypeptides" are polypeptides with the ability to emit fluorescence. In the present optical probe, an optically active polypeptide, such as a fluorescent protein, is operably inserted into a arginine-sensitive polypeptide. As used herein, the term "fluorescence property" refers to the molar extinction coefficient at an appropriate excitation wavelength, the fluorescence quantum efficiency, the shape of the excitation spectrum or the emission spectrum, the excitation wavelength maximum and the emission wavelength maximum, the amplitude of two different wavelength excitations, the emission amplitude ratio of two different wavelengths, the excited state lifetime or the fluorescence anisotropy. The measurable difference in any of these properties between the active and inactive states is sufficient for the utility of the fluorescent protein substrate of the present invention in activity assays. The measurable difference can be determined by determining the amount of any quantitative fluorescent property, for example, the amount of fluorescence at a specific wavelength or the integral of fluorescence on the emission spectrum. Preferably, the protein substrate is selected to have fluorescence properties that are easily distinguishable in the unactivated and activated conformational states. The optically active polypeptide described herein may also include a functional variant thereof. A functional variant of the optically active polypeptide includes, but is not limited to, a variant for which the same or similar change in fluorescence property may occur as the parent optically active polypeptide.

A "linker" or "linker region" refers to an amino acid or nucleotide sequence linking two parts in a polypeptide, protein, or nucleic acid of the invention. Exemplarily, the number of amino acids at the amino terminus of the linker region of the fructose-1,6-bisphosphate sensitive polypeptide to the optically active polypeptide in the invention is selected to be 0-3, and the number of amino acids at the carboxyl terminus is selected to be 0-2; when a recombinant optical probe is linked to a functional protein as a basic unit, it can be fused at the amino or carboxyl terminus of the recombinant optical probe. The linker sequence may be a short peptide chain composed of one or more flexible amino acids, as in Y.

The term "fluorescent protein" used herein refers to a protein that fluoresces under irradiation of excitation light. Fluorescent proteins have been used as basic detection means in the field of biological sciences, such as green fluorescent protein (GFP) commonly used in biotechnology and circularly rearranged blue fluorescent protein (cpBFP), circular rearranged green fluorescent protein (cpGFP), circularly rearranged yellow fluorescent protein (cpYFP) derived from mutations in this protein, etc; there is also a red fluorescent protein (RFP) commonly used in the art, and circular rearranged proteins derived from this protein, such as cpmApple, cpmOrange, cpmKate, etc. Exemplarily, cpYFP is shown in SEQ ID NO: 2, cpmOrange is shown in SEQ ID NO: 3, cpmKate is shown in SEQ ID NO: 4 or 8, mCherry is shown in SEQ ID NO: 5, cpGFP is shown in SEQ ID NO: 6, cpBFP is shown in SEQ ID NO: 7, and cpmApple is shown in SEQ ID NO: 9.

The fluorescent protein in the optical probe also includes functional variants with mutations, including but not limited to fluorescent proteins with mutations in amino acids at position 1-3 (preferably at position 1), such as mutations to V or I. Exemplarily, the functional variant of cpYFP has a sequence as shown in SEQ ID NO: 2 and has a mutation V or I at the Y1 position.

In the optical probe of the present invention, the optically active polypeptide is located between residues 174-185 and/or 201-208 of the fructose-1,6-bisphosphate sensitive polypeptide in the N-C direction, and numbered corresponding to the full length of the fructose-1,6-bisphosphate sensitive polypeptide.

Exemplarily, the optically active polypeptide is located at the following positions of the amino acid sequence of fructose-1,6-bisphosphate binding protein: 174/175, 174/176, 174/177, 174/178, 174/179, 174/180, 174/181, 174/182, 174/183, 174/184, 174/185, 175/176, 175/177, 175/178, 175/179, 175/180, 175/181, 175/182, 175/183, 175/184, 175/185 75/185, 176/177, 176/178, 176/179, 176/180, 176/181, 176/182, 176/183, 176/184, 176/185, 177/178, 177/179, 177/180, 177/181, 177/182, 177/183, 177/184, 177/185, 178/179, 178/180, 178/181, 178/182, 178/183, 178/184, 178/185, 179/ 180, 179/181, 179/182, 179/183, 179/184, 179/185, 180/181, 180/182, 180/183, 180/184, 180/185, 181/182, 181/182, 181/183, 181/184, 181/185, 182/183, 182/184, 182/185, 183/184, 183/185, 184/185, 201/202, 201/203, 201/204, 201/205 , 201/206, 201/207, 201/208, 202/203, 202/204, 202/205, 202/206, 202/207, 202/208, 203/204, 203/205, 203/206, 203/207, 203/208, 204/205, 204/206, 204/207, 204/208, 205/206, 205/206, 205/207, 205/208, 206/207, 206/208, 207/208. In the present invention, if the two numbers in a position represented in "X/Y" form are consecutive integers, it means that the optically active polypeptide is located between the amino acids stated by those numbers. For example, insertion position 147/148 indicates that the optically active polypeptide is located between amino acids 147 and 148 of the fructose-1,6-bisphosphate sensitive polypeptide. If the two numbers in a position represented in "X/Y" form are not consecutive integers, it means that the optically active polypeptide replaces the amino acids between the amino acids indicated by the numbers. For example, insertion position 174/185 indicates that the optically active polypeptide replaces the amino acids 175-184 of the fructose-1,6-bisphosphate sensitive polypeptide. Preferably, the optically active polypeptide is inserted into the following positions of the fructose-1,6-bisphosphate-sensitive polypeptide: 176/177, 176/178, 177/181, 179/180, 182/184, 182/185, 203/206, 203/207 and 203/208.

In an exemplary embodiment, the B1-A-B2 optical probe of the present invention may be a probe formed when a fluorescent protein is located at 176/177, 176/178, 177/181, 179/180, 182/184, 182/185, 203/206, 203/207 and 203/208 of CggR or a truncated variant thereof (e.g., SEQ ID NO: 1 or a fragment thereof comprising amino acids 88-340). In a specific embodiment, the fructose-1,6-bisphosphate-sensitive polypeptide in the optical probe is as shown in amino acids 88-340 of SEQ ID NO: 1, the optically active polypeptide is as shown in any one of SEQ ID NO: 2-9, and the optically active polypeptide is located at the following positions of the fructose-1,6-bisphosphate-sensitive polypeptide: 176/177, 176/178, 177/181, 179/180, 182/184, 182/185, 203/206, 203/207 or 203/208.

When referring to a certain polypeptide or protein, the term "variant" or "mutant" used herein includes variants that have the same function of the polypeptide or protein but have different sequence. Variants of polypeptides or proteins can include: homologous sequences, conservative variants, allelic variants, natural mutants, induced mutants. These variants include, but are not limited to: deletions, insertions and/or substitutions of one or more (usually 1-30, preferably 1-20, more preferably 1-10, most preferably 1-5) amino acids in the sequence of the polypeptide or protein, and sequences obtained by adding one or more (usually within 20, preferably within 10, more preferably within 5) amino acids to its carboxy terminus and/or amino terminus. These variants may also comprise at least about 70%, at least about 75%, at least about 80%, at least about 85%, at least about 90%, at least about 95%, at least about 98%, at least about 99% or 100% sequence identity to the polypeptide or protein. Not limited by theory, an amino acid residue is altered without changing the overall configuration and function of the polypeptide or protein, i.e., a functionally conserved mutation. For example, in the art, substitution with amino acids of approaching or similar perperty generally does not change the function of a polypeptide or protein. In the art, amino acids with similar property tend to refer to families of amino acids with similar side chains, which have been well defined in the art. These families include amino acids with basic side chain (e.g., lysine, arginine, histidine), amino acids with acidic side chain (e.g., aspartic acid, glutamic acid), amino acids with uncharged polar side chain (e.g., glycine, asparagine, glutamine, serine, threonine, tyrosine, cysteine), amino acids with non-polar side chain (e.g., alanine, valine, leucine, isoleucine, arginine, phenylalanine, methionine, tryptophan), amino acids with β-branched side chain (e.g., threonine, valine, isoleucine) and amino acids with aromatic side chain (e.g., tyrosine, phenylalanine, tryptophan, histidine). For another example, the addition of one or more amino acids to the amino terminus and/or carboxy terminus also generally does not alter the function of a polypeptide or protein. The conservative amino acid substitutions of many commonly known non-genetic coding amino acids are known in the art. Conservative substitutions of other non-coding amino acids can be determined based on a comparison of their physical properties with those of the amino acids that are genetically encoded.

The inventors discovered that a fructose-1,6-bisphosphate binding protein variant having a mutation at a position selected from the following positions of SEQ ID NO: 1 or a truncated variant thereof exhibits a binding activity different from fructose-1,6-bisphosphate: T148, R175, G176, G177, L178, E180, D181, V182, K183, N184, Q185, F271. The amino acid mutation includes modification, substitution or deletion of an amino acid. In particular, a fructose-1,6-bisphosphate binding protein variant having a mutation at one, two or three, four or five positions selected from any one of the following groups of SEQ ID NO: 1 or its truncated variants is more advantageous for use in the optical probe of the present invention to more efficiently detect fructose-1,6-bisphosphate: (a) T148, R175, G176, G177, L178, D181, V182, K183, N184, Q185, (b) G177, L178, E180, D181, V182, N184, Q185, F271. In a preferred embodiment, the mutation of the fructose-1,6-bisphosphate binding protein variant includes mutations at positions selected from any one of the following groups: (1) G177 and L178, (5) D181 and V182, (6) E180 and D181, (7) N184 and Q185; or, the mutation comprises a mutation at a position selected from any one of the following groups: (1) G177, L178, (2) G177, L178 and Q185, (3 )G177, L178 and T148, (4) G177, L178 and F271, (5) D181, V182, (6) E180, D181, (7) N184, Q185, (8) N184, Q185, D181, V182, G177, (9) N184, Q185, G177, (10) N184, Q185, D181, V182.

Wherein, as an example in the embodiment, in SEQ ID NO: 1 or its truncated variant, T148 is mutated to S; G177 is mutated to A, I, L, M or N, preferably is mutated to L or M; L178 is mutated to Y, F or N, preferably is mutated to N; E180 is mutated to G; D181 is mutated to F, L or E, preferably is mutated to E; V182 is mutated to F, L or G, preferably is mutated to G; N184 is mutated to V; Q185 is mutated to S, A, T, D or P, preferably is mutated to P; F271 is mutated to Y.

In a preferred embodiment, the mutation for the fructose-1,6-bisphosphate binding protein variant (SEQ ID NO: 1 or a truncated variant thereof) is selected from any one of the following: (1) G177A and L178Y, (2) G177I and L178F, (3) G177L and L178Y, (4) G177M and L178N, (5) G177N and L178Y, (6) G177M, L178N and Q185S, (7) G177M, L178N and Q185A, (8) G177M, L178N and Q185T, (9) G177M, L178N and Q185D, (10) G177M, L178N and T148S, (11) G177M, L178N and F271Y, (12) D181F and V182F, (13) D181L and V182L, (14) E180G and D181G, (15) N184V and Q185P, (16) N184V, Q185P, D181E, V182G and G177L, (17) N184V, Q185P and G177L, (18) N184V, Q185P, D181E and V182G.

The present invention provides fructose-1,6-bisphosphate binding protein variants having these mutations and optical probes comprising such fructose-1,6-bisphosphate binding protein variants as fructose-1,6-bisphosphate sensitive polypeptides. Therefore, in one or more embodiments, the fructose-1,6-bisphosphate sensitive polypeptide in the optical probe is a fructose-1,6-bisphosphate binding protein variant as described in any embodiment of the present invention, and the fluorescent protein in the optical probe is as shown in SEQ ID NO: 2-9 and the first amino acid of the fluorescent protein is mutated to I or V.

In some specific embodiments, the fructose-1,6-bisphosphate-sensitive polypeptide in the optical probe is as shown in amino acids 88-340 of SEQ ID NO: 1, the optically active polypeptide is as shown in SEQ ID NO: 2, the optically active polypeptide is located at position 176/177 of the fructose-1,6-bisphosphate sensitive polypeptide, and the fructose-1,6-bisphosphate-sensitive polypeptide has a mutation selected from any one of the following: (1) G177A and L178Y, (2) G177I and L178F, (3) G177L and L178Y, (4) G177M and L178N, (5)177N and L178Y, (6) G177M, L178N and Q185S, (7) G177M, L178N and Q185A, (8) G177M, L178N and Q185T, (9) G177M, L178N and Q185D, (10) G177M, L178N and T148S, (11) G177M, L178N and F271Y. Exemplarily, the amino acid sequence of the optical probe shown in item (4) is shown in SEQ ID NO: 10, and the nucleic acid sequence is shown in SEQ ID NO: 17; the amino acid sequence of the optical probe shown in item (7) is shown in SEQ ID NO: 11, and the nucleic acid sequence is shown in SEQ ID NO: 18; the amino acid sequence of the optical probe shown in item (8) is shown in SEQ ID NO: 12, and the nucleic acid sequence is shown in SEQ ID NO: 19; the amino acid sequence of the optical probe shown in item (9) is shown in SEQ ID NO: 13, and the nucleic acid sequence is shown in SEQ ID NO: 20.

In some specific embodiments, the fructose-1,6-bisphosphate-sensitive polypeptide in the optical probe is as shown in amino acids 88-340 of SEQ ID NO: 1, the optically active polypeptide is as shown in SEQ ID NO: 2, the optically active polypeptide is located at the 177/181 position of the fructose-1,6-bisphosphate sensitive polypeptide, and the fructose-1,6-bisphosphate sensitive polypeptide has a mutation selected from any one of the following: (12) D181F and V182F, (13) D181L and V182L.

In some specific embodiments, the fructose-1,6-bisphosphate-sensitive polypeptide in the optical probe is as shown in amino acids 88-340 of SEQ ID NO: 1, the optically active polypeptide is as shown in SEQ ID NO: 2, the optically active polypeptide is located at the 179/180 position of the fructose-1,6-bisphosphate sensitive polypeptide, and the fructose-1,6-bisphosphate sensitive polypeptide has a mutation selected from any one of the following: (14) E180G and D181G.

In some specific embodiments, the fructose-1,6-bisphosphate-sensitive polypeptide in the optical probe is as shown in amino acids 88-340 of SEQ ID NO: 1, the optically active polypeptide is as shown in SEQ ID NO: 2, the optically active polypeptide is located at position 182/184 of the fructose-1,6-bisphosphate-sensitive polypeptide, and the optical probe has a mutation selected from any one of the following:(15) N184V and Q185P of the fructose-1,6-bisphosphate-sensitive polypeptide, (16) N184V, Q185P, D181E, V182G, G177L of the fructose-1,6-bisphosphate-sensitive polypeptide, and the first amino acid of the optically active polypeptide is mutated to V, (17) N184V, Q185P, D181E, V182G, G177L of the fructose-1,6-bisphosphate-sensitive polypeptide 82G, G177L, and the first amino acid of the optically active polypeptide mutated to I, (18) N184V, Q185P and G177L of a fructose-1,6-bisphosphate-sensitive polypeptide, and the first amino acid of the optically active polypeptide mutated to V, (19) N184V, Q185P and G177L of a fructose-1,6-bisphosphate-sensitive polypeptide, and the first amino acid of the optically active polypeptide mutated to I, (20) N184V, Q185P, D181E and V182G of a fructose-1,6-bisphosphate-sensitive polypeptide, and the first amino acid of the optically active polypeptide mutated to V. Exemplarily, the amino acid sequence of the optical probe shown in item (16) is shown in SEQ ID NO: 14, and the nucleic acid sequence is shown in SEQ ID NO: 21; the amino acid sequence of the optical probe shown in item (17) is shown in SEQ ID NO: 15, and the nucleic acid sequence is shown in SEQ ID NO: 22; the amino acid sequence of the optical probe shown in item (20) is shown in SEQ ID NO: 16, and the nucleic acid sequence is shown in SEQ ID NO: 23.

In two or more sequences of a polypeptide or nucleic acid molecule, the terms "identity" or "percent identity" refer to when the maximum correspondence is compared and aligned by manual alignment and visual inspection using methods known in the art such as sequence comparison algorithms, in a comparison window or a specified region, two or more sequences or subsequences are identical or have a certain percentage of amino acid residues or nucleotides that are identical in the specified region (e.g., 60%, 65%, 70%, 75%, 80%, 85%, 90%, 91%, 92%, 93%, 94%, 95%, 96%, 97%, 98%, 99%, or 100% identical). For example, the preferred algorithms suitable for determination of percent identity and percent similarity are the BLAST and BLAST 2.0 algorithms, respectively, see Altschul et al., (1977) nucleic acids res. 25:3389 and Altschul et al., (1990) J. mol. Biol 215:403.

Those skilled in the art is well known that in gene cloning operation, it is often necessary to design a suitable restriction enzyme cutting position, which is bound to introduce one or more incoherent residues at the ends of the expressed polypeptide or protein, and this does not affect the activity of the polypeptide or protein of interest. Also for constructing fusion proteins, facilitating the expression of recombinant proteins, obtaining recombinant proteins that are automatically secreted outside the host cell, or facilitating the purification of recombinant proteins, it is often necessary to add some amino acids to the N-terminus, C-terminus, or other suitable regions within the recombinant protein, for example, including but not limited to, suitable linker peptides, signal peptides, leader peptides, terminal extensions, glutathione S-transferases (GSTs) maltose E-binding protein, Protein A, tags such as 6His or flag, or the proteolytic enzyme positions of Factor Xa or thrombin or enterokinase.

The terms "functional fragment", "derivative", and "analog" used herein refer to proteins that maintain essentially the same biological function or activity as the original polypeptide or protein (e.g., a fructose-1,6-bisphosphate binding protein or a fluorescent protein). Functional variants, derivatives, or analogues of a polypeptide or protein (e.g., a fructose-1,6-bisphosphate binding protein or a fluorescent protein) of the invention may be (i) a protein having one or more conservative or nonconservative amino acid residues (preferably conservative amino acid residues) substituted, whereas such substituted amino acid residues may or may not be encoded by the genetic code, or (ii) a protein having a substituted group in one or more amino acid residues, or (iii) a protein formed by the fusion of the mature protein to another compound, such as a compound that extends the half-life of the protein, such as polyethylene glycol, or (iv) a protein formed by the fusion of an additional amino acid sequence to this protein sequence (such as a secretory sequence or the sequence or protein used to purify this protein, or the fusion protein formed with an antigenic IgG fragment). According to the teaching herein, these functional variants, derivatives, and analogues belong to the common knowledge to those skilled in the art. The analogues also include analogues with a residue (such as a D-amino acid) different from the natural L-amino acid, as well as those with non-natural or synthetic amino acids (such as β, γ-amino acids). It is understood that the fructose-1,6-bisphosphate sensitive polypeptides of the present invention are not limited to the representative proteins, variants, derivatives, and analogues above. Modified (usually without changing the primary structure) forms include chemically derived forms of the protein such as acetylated or carboxylated forms, in vivo or in vitro. Modifications also include glycosylation, such as those resulting from glycosylation modifications either in the synthesis and processing of the protein or in further processing steps. This modification can be accomplished by exposing the protein to enzymes for glycosylation, such as mammalian glycosylases or deglycosylases. The modified forms also include sequences with phosphorylated amino acid residues such as phosphotyrosine, phosphoserine, phosphothreonine. It also includes proteins that have been modified to improve their anti-proteolytic properties or optimize solubility.

The present fusion polypeptide comprises the optical probe described herein and an additional polypeptide. In some embodiments, the optical probe described herein also comprises additional polypeptide fused thereto. The additional polypeptide described herein does not affect the property of the optical probe. The additional polypeptide may be located N- and/or C-terminal of the optical probe. In some embodiments, other polypeptides include polypeptides that localize optical probes to different organelles or subcellular organelles, tags for purification, or tags for immunoblotting. There may be a linker between the optical probe and the additional polypeptide in the fusion polypeptide described herein.

The subcellular organelles described here include cytoplasm, mitochondria, nucleus, endoplasmic reticulum, cell membrane, Golgi apparatus, lysosome and peroxisome, etc. In some embodiments, the tags for purification or the tags for immunoblotting include 6 histidine (6*His), glutathione sulfurtransferase (GST), Flag.

The present invention includes nucleic acid molecules encoding the fructose-1,6-bisphosphate sensitive polypeptide or optical probe of the present invention. The terms "nucleic acid" or "nucleotide" or "polynucleotide" or "nucleic acid sequence" used herein may be in the form of DNA or in the form of RNA. DNAs include cDNAs, genomic DNAs, or synthetic DNAs. DNAs can be single stranded DNAs or double stranded DNAs. DNAs can be coding or noncoding strand DNAs. When referring to nucleic acids, the term "variant" used herein may be a naturally occurring allelic variant or a non-naturally occurring variant. These nucleotide variants include degenerate variants, substitution variants, deletion variants, and insertion variants. As is known in the art, an allelic variant is a form of substitution of a nucleic acid, which may be a substitution, deletion, or insertion of one or more nucleotides but does not substantially alter the function of the protein it encodes. The nucleic acid of the invention may comprise a nucleotide sequence having sequence identity of at least about 50%, at least about 60%, at least about 70%, at least about 75%, at least about 80%, at least about 85%, at least about 90%, at least about 95%, at least about 98%, at least about 99%, or 100% to the nucleic acid sequence. The invention also relates to a nucleic acid fragment that hybridizes to the sequences described above. As used herein, a "nucleic acid fragment" is at least 15 nucleotides in length, preferably at least 30 nucleotides, more preferably at least 50 nucleotides, and even more preferably at least 100 nucleotides or more. The nucleic acid fragment can be used in amplification techniques of nucleic acids (such as PCR).

The full-length sequences or fragments thereof of the optical probes or fusion proteins of the invention can often be obtained by PCR amplification, artificial synthesis or recombinantion. Those skilled in the art are aware of the steps and reagents used in conventional PCR, synthesis and recombination methods. In addition, mutations can be introduced into the protein sequence of the invention by methods such as mutation PCR or chemical synthesis.

The present invention also relates to a nucleic acid construct containing the polynucleotides described herein, and one or more regulatory sequences operably linked to such sequences. The polynucleotides described herein can be manipulated in a variety of ways to guarantee expression of the polypeptide or protein. The nucleic acid construct can be manipulated according to the difference or requirements of the expression vector prior to insertion of the nucleic acid construct into the vector. Techniques that utilize recombinant DNA methods to alter polynucleotide sequences are known in the art.

In certain embodiments, the nucleic acid construct is a vector. The vector can be a cloning vector, an expression vector, or a homologous recombination vector. The polynucleotides of the present description can be cloned into many types of vectors, e.g., plasmids, phagemids, phage derivatives, animal viruses, and cosmids.

A typical expression vector contains expression control sequences that can be used to regulate the expression of the desired nucleic acid sequence, and is operably connected to the nucleic acid sequence of the present invention or its complementary sequence. The term "expression control sequence" used herein refers to elements that regulate the transcription, translation, and expression of a target gene and can be operably linked to the gene of interest, which may be a replication origin, promoter, marker gene, or translational control element, including enhancers, operons, terminators, ribosome binding positions, etc., and the choice of expression control sequence depends on the host cell used. In a recombinant expression vector, "operable ligation" refers to the attachment of the nucleotide sequence of interest to a regulatory sequence in a manner that allows the expression of the nucleotide sequence. Those skilled in the art are well known of methods for constructing expression vectors containing the present fusion protein coding sequences and suitable transcription/translation control signals. These methods include in vitro recombinant DNA techniques, DNA synthesis techniques, in vivo recombination techniques, etc. The DNA sequence can be effectively ligated to an appropriate promoter in an expression vector to direct mRNA synthesis. Representative examples of these promoters are: the lac or trp promoter of E. coli; λ-phage PL promoter; eukaryotic promoters including the CMV immediate early promoter, the HSV thymidine kinase promoter, the early and late SV40 promoters, the LTR of retroviruses, and several other promoters known to control gene expression in prokaryotic or eukaryotic cells or their viruses. The expression vector also includes a ribosome binding position for translation initiation and a transcription terminator. In one embodiment, the expression vector may use a commercially available pCDF vector without other special requirements. Exemplarily, BamHI and EcoRI were employed to double digest the nucleotide sequence encoding the optical probe and the expression vector, respectively, and then the enzymatic cleavage products of both were ligated to give recombinant expression vectors. The present invention makes no special limitation to the specific steps and parameters of enzymatic cleavage and ligation, and those conventional in the art would work.

After a recombinant expression vector is obtained, this vector is transformed into host cells to produce a protein or peptide including the fusion protein. This transfer process can be performed with conventional techniques well known to those skilled in the art, such as transformation or transfection. The host cells of the present invention refer to cells capable of receiving and accommodating recombinant DNA molecules, which are positions of recombinant gene amplification, and ideally the recipient cell should satisfy both conditions of easy access and proliferation. The "host cells" of the invention may include prokaryotic cells and eukaryotic cells, specifically including bacterial cells, yeast cells, insect cells, and mammalian cells. The host cells are preferably a variety of cells favouring the expression or fermentative production of the gene product, such cells being well known and commonly used in the art. Specifically, it can be bacterial cells of Escherichia coli, Streptomyces, Salmonella typhimurium, fungal cells such as yeast, plant cells, insect cells of Drosophila S2 or Sf9, CHO, COS, HEK293, HeLa cells, or animal cells of Bowes melanoma cells, etc. The exemplary host cell used in the embodiments of the present invention is the Escherichia coli BL21-DE3 strain. Those skilled in the art well know how to select appropriate vectors, promoters, enhancers, and host cells.

The methods of transfer to host cells described herein are conventional methods in the art, including calcium phosphate or calcium chloride coprecipitation, DEAE-mannan-mediated transfection, lipofection, naturally competent cells, chemically mediated transfer, or electroporation. When the host is a prokaryote such as E. coli, the process described is preferably CaCl₂ method or MgCl₂ method, and their steps used are well known in the art. When the host cell is a eukaryotic cell, the following DNA transfection methods are used: calcium phosphate coprecipitation, conventional mechanical methods such as microinjection, electroporation, liposome packaging, etc.

After the expression vector is transferred into the host cell, the host cell into which the expression vector is transferred is cultured for amplification and expression,and then separat and obtain fructose-1,6-bisphosphate optical probe. The host cells are expanded for expression by conventional methods. Depending on the host cell species used, the medium used in culture may be various conventional media. Culture is performed under conditions suitable for host cell growth.

In the present invention, an optical probe is expressed intracellularly, on the cell membrane, or secreted outside the cell. If necessary, the recombinant protein can be separated or purified by various separation methods using its physical, chemical and other properties. The present invention does not specifically limit the method for separating the fructose-1,6-bisphosphate fluorescent protein, and the conventional separation method of the fusion protein in the art can be used. These methods are well known to those skilled in the art and include, but are not limited to: conventional renaturation treatments, salting out methods, centrifugation, osmotic disruption, sonication, ultra centrifugation, molecular sieving chromatography, adsorption chromatography, ion exchange chromatography, high performance liquid chromatography (HPLC) and other liquid chromatography techniques and the combination of these methods. In one embodiment, His tag affinity chromatography is utilized for optical probe isolation.

**The invention also provides use of fructose-1,6-bisphosphate optical probes in real-time localization, quantitative detection and high-throughput compound screening of fructose-1,6-bisphosphate.** In one aspect, the fructose-1,6-bisphosphate optical probe, preferably linked with signal peptides at different parts of the cell, is transferred into cells for real-time localization of fructose-1,6-bisphosphate by detecting the strength of fluorescence signals in cells; corresponding quantitative detection of fructose-1,6-bisphosphate is performed by a fructose-1,6-bisphosphate standard titration curve. The change of the fluorescence signal is displayed by, for example, a standardized fluorescence signal ratio. In the embodiment involving cpYFP, the ratio is the ratio of the ratio of the sample's 485-nanometer fluorescence signal to the 420-nanometer fluorescence signal to the corresponding ratio of the control. The fructose-1,6-bisphosphate standard titration curve of the present invention is drawn based on the fluorescence signal of the fructose-1,6-bisphosphate optical probe under different concentrations of fructose-1,6-bisphosphate. The fructose-1,6-bisphosphate optical probe described herein is directly transferred into cells, which is more accurate without time-consuming sample processing process during the real-time localization and quantitative detection of fructose-1,6-bisphosphate. When the fructose-1,6-bisphosphate optical probe of the present invention is used for high-throughput compound screening, different compounds are added to the cell culture fluid, and the change of fructose-1,6-bisphosphate content is measured, so as to screen out compounds that affect the change of fructose-1,6-bisphosphate content. The application of the fructose-1,6-bisphosphate optical probe of the present invention in real-time localization, quantitative detection and high-throughput compound screening of fructose-1,6-bisphosphate is for non-diagnostic and therapeutic purposes, and does not involve the diagnosis and treatment of diseases.

The present invention also provides a detection kit comprising the optical probe, nucleic acid molecule, nucleic acid construct and/or cell of the present invention. The kit also contains other reagents required for detecting fructose-1,6-bisphosphate. The other reagents are well known in the art, such as buffer, cell culture medium, fructose-1,6-bisphosphate standard. Exemplary buffers are, for example, 100 mM HEPES and 100 mM NaCl, pH 7.4.

Concentrations, contents, percentages, and other values may be expressed with ranges available herein. It is also understood that use of these ranges is only for convenience and conciseness, which should be interpreted elastically to include values explicitly mentioned in the upper and lower limits of the range, but also to include all individual values or sub ranges included in the ranges.

### Some specific embodiments

Item 1. A fructose-1,6-bisphosphate binding protein variant, which:
(1) having a sequence as shown in SEQ ID NO: 1 and having a mutation at one, two, three, four, five or more positions selected from the group consisting of:T148, R175, G176, G177, L178, E180, D181, V182, K183, N184, Q185, F271, and wherein the mutation comprises a modification, substitution or deletion of an amino acid,
(2) is a truncated variant of (1) having amino acids 88 to 340, or
(3) is a sequence having at least 70% sequence identity with the sequence of (1) or (2) and having the mutation described in (1) and retaining the ability to bind to fructose-1,6-bisphosphate,

preferably, the mutated positions are selected from 1, 2 or 3, 4, or 5 positions of any one of the following groups: (a) T148, R175, G176, G177, L178, D181, V182, K183, N184, Q185, (b) G177, L178, E180, D181, V182, N184, Q185, F271,
more preferably, the mutation comprises mutations at positions selected from any one of the following groups: (1) G177 and L178, (5) D181 and V182, (6) E180 and D181, (7) N184 and Q185; or, the mutation comprises a mutation at positions selected from any one of the following groups: (1) G177, L178, (2) G177, L178 and Q185, (3 )G177, L178 and T148, (4) G177, L178 and F271, (5) D181, V182, (6) E180, D181, (7) N184, Q185, (8) N184, Q185, D181, V182, G177, (9) N184, Q185, G177, (10) N184, Q185, D181, V182,
more preferably, T148 is mutated to S; G177 is mutated to A, I, L, M or N, preferably is mutated to L or M; L178 is mutated to Y, F or N, preferably is mutated to N; E180 is mutated to G; D181 is mutated to F, L or E, preferably is mutated to E; V182 is mutated to F, L or G, preferably is mutated to G; N184 is mutated to V; Q185 is mutated to S, A, T, D or P, preferably is mutated to P; F271 is mutated to Y.
more preferably, the mutation comprises mutations selected from any one of the following groups: (1) G177A and L178Y, (2) G177I and L178F, (3) G177L and L178Y, (4) G177M and L178N, (5) G177N and L178Y, (6) G177M, L178N and Q185S, (7) G177M, L178N and Q185A, (8) G177M, L178N and Q185T, (9) G177M, L178N and Q185D, (10) G177, L178 and T148S, (11) G177, L178 and F271Y, (12) D181F and V182F, (13) D181L and V182L, (14) E180G and D181G, (15) N184V and Q185P, (16) N184V, Q185P, D181E, V182G and G177L, (17) N184V, Q185P and G177L, (18) N184V, Q185P, D181E and V182G.

Item 2. A fructose-1,6-bisphosphate optical probe, comprising a fructose-1,6-bisphosphate sensitive polypeptide and an optically active polypeptide, wherein the optically active polypeptide is located within the sequence of the fructose-1,6-bisphosphate sensitive polypeptide, the fructose-1,6-bisphosphate sensitive polypeptide is a fructose-1,6-bisphosphate binding protein or a functional variant thereof, and the optically active polypeptide is a fluorescent protein or a functional variant thereof; wherein the functional variant of the fructose-1,6-bisphosphate binding protein has a mutation within 7 amino acids from the connection with the optically active polypeptide, and the functional variant of the fluorescent protein has a mutation within 3 amino acids from the connection with the optically active polypeptide.

Item 3. An optical probe as described in Item 1, wherein the fructose-1,6-bisphosphate binding protein has the sequence shown in SEQ ID NO: 1 or a truncation having amino acids 88-340 thereof, or a sequence having at least 70% sequence identity therewith and retaining sensitivity to fructose-1,6-bisphosphate.

Item 4. The optical probe of Item 1, wherein the optically active polypeptide is located between residues 174-185 and/or 201-208 of the fructose-1,6-bisphosphate sensitive polypeptide,
preferably, the optically active polypeptide is located at any one or more of the following positions of the fructose-1,6-bisphosphate sensitive polypeptide: 174/175, 174/176, 174/177, 174/178, 174/179, 174/180, 174/181, 174/182, 174/183, 174/184, 174/185, 175/176, 175/177, 175/178, 175/179, 175/180, 175/181, 175/182, 175/183, 175/184, 175/185 75/185, 176/177, 176/178, 176/179, 176/180, 176/181, 176/182, 176/183, 176/184, 176/185, 177/178, 177/179, 177/180, 177/181, 177/182, 177/183, 177/184, 177/185, 178/179, 178/180, 178/181, 178/182, 178/183, 178/184, 178/185, 179/ 180, 179/181, 179/182, 179/183, 179/184, 179/185, 180/181, 180/182, 180/183, 180/184, 180/185, 181/182, 181/182, 181/183, 181/184, 181/185, 182/183, 182/184, 182/185, 183/184, 183/185, 184/185, 201/202, 201/203, 201/204, 201/205 , 201/206, 201/207, 201/208, 202/203, 202/204, 202/205, 202/206, 202/207, 202/208, 203/204, 203/205, 203/206, 203/207, 203/208, 204/205, 204/206, 204/207, 204/208, 205/206, 205/206, 205/207, 205/208, 206/207, 206/208 and/or 207/208, more preferably, the optically active polypeptide is located at any one or more of the following positions of the fructose-1,6-bisphosphate-sensitive polypeptide: 176/177, 176/178, 177/181, 179/180, 182/184, 182/185, 203/206, 203/207 and 203/208;
preferably, the functional variant of fructose-1,6-bisphosphate binding protein is as described in claim 1;
preferably, the fluorescent protein is selected from yellow fluorescent protein, orange fluorescent protein, red fluorescent protein, green fluorescent protein, blue fluorescent protein, apple red fluorescent protein, and the functional variant of the fluorescent protein has a mutation at the amino acid 1-3; more preferably, the fluorescent protein has a sequence shown in any one of SEQ ID NOs: 2-9, and the functional variant of the fluorescent protein has a mutation at the first amino acid,
preferably, the functional variant of the fluorescent protein comprises a mutation in which the first amino acid of the fluorescent protein is mutated to I or V,
preferably, the fructose-1,6-bisphosphate sensitive polypeptide is a truncated variant of SEQ ID NO: 1 having amino acids 88-340, and the optically active polypeptide is located at any one or more of the following positions of the fructose-1,6-bisphosphate sensitive polypeptide: 176/177, 176/178, 177/181, 179/180, 182/184, 182/185, 203/206, 203/207 and 203/208; more preferably, the optical probe has a sequence shown in any one of SEQ ID NO: 10-18,
preferably, the fructose-1,6-bisphosphate sensitive polypeptide is a truncated variant of SEQ ID NO: 1 having amino acids 88-340, the optically active polypeptide is located at any one or more of the following positions of the fructose-1,6-bisphosphate sensitive polypeptide: 176/177, 177/181, 179/180 or 182/184, and the optical probe has one or more mutations selected from the group consisting of: T148S, G177A, G1771, G177L, G177 M, G177N, L178Y, L178F, L178Y, L178N, L178Y, L178N, E180G, D181E, D181F, D181L, D181G, V182F, V182L, V182G, N184V, Q185A, Q185S, Q185A, Q185T, Q185D, Q185P, F271Y, the first amino acid of the optically active polypeptide is mutated to V or I,
preferably, the optical probe has a mutation selected from any one of the following groups: (1) G177A and L178Y of the fructose-1,6-bisphosphate sensitive polypeptide, (2) G177I and L178F of the fructose-1,6-bisphosphate sensitive polypeptide, (3) G177L and L178Y of the fructose-1,6-bisphosphate sensitive polypeptide, (4) G177M and L178N of the fructose-1,6-bisphosphate sensitive polypeptide, (5) G177N and L178Y of the fructose-1,6-bisphosphate sensitive polypeptide, (6) G177M, L178N and Q185S of the fructose-1,6-bisphosphate sensitive polypeptide, (7) G177M, L178N and Q185A of the fructose-1,6-bisphosphate sensitive polypeptide, (8) G177M, L178N and Q185T of the fructose-1,6-bisphosphate sensitive polypeptide, (9) G177M, L178N and Q185D of the fructose-1,6-bisphosphate sensitive polypeptide, (10) G177, L178 and T148S of the fructose-1,6-bisphosphate sensitive polypeptide, (11) G177, L178 and F271Y of the fructose-1,6-bisphosphate sensitive polypeptide, (12) D181F and V182F of the fructose-1,6-bisphosphate sensitive polypeptide, (13) D181L and V182L of the fructose-1,6-bisphosphate sensitive polypeptide, (14) E180G and D181G of the fructose-1,6-bisphosphate sensitive polypeptide, (15) N184V and Q185P of the fructose-1,6-bisphosphate sensitive polypeptide, (16) N184V, Q185P, D181E, V182G, G177L of the fructose-1,6-bisphosphate sensitive polypeptide, and the first amino acid of the optically active polypeptide is mutated to V, (17) N184V, Q185P, D181E,V182G,G177L of the fructose-1,6-bisphosphate sensitive polypeptide and the first amino acid of the optically active polypeptide is mutated to I, (18) N184V, Q185P and G177L of the fructose-1,6-bisphosphate sensitive polypeptide, and the first amino acid of the optically active polypeptide is mutated to V, (19) N184V, Q185P and G177L of the fructose-1,6-bisphosphate sensitive polypeptide, and the first amino acid of the optically active polypeptide is mutated to I, (20) N184V, Q185P, D181E and V182G of the fructose-1,6-bisphosphate sensitive polypeptide, and the first amino acid of the optically active polypeptide is mutated to V.

Item 5. A nucleic acid molecule comprising:
(a) a coding sequence of the optical probe described in any one of items 2 to 4,
(b) a complement sequence of (a).

Item 6. A nucleic acid construct comprising the nucleic acid molecule described in Item 5,
preferably, the nucleic acid construct is a cloning vector, an expression vector or a recombinant vector.

Item 7. A host cell, wherein the host cell:
(1) expresses the optical probe according to any one of items 2 to 4;
(2) comprises the nucleic acid molecule according to Item 5; and/or
(3) comprises the nucleic acid construct according to Item 6.

Item 8. A detection kit comprising:
(1) the optical probe according to any one of items 2 to 4,
(2) the nucleic acid sequence according to item 5,
(3) the nucleic acid construct according to item 6, or
(4) the host cell according to item 7,

the detection kit optionally further comprises other reagents required for detecting fructose-1,6-bisphosphate using an optical probe,
preferably, the detection kit further comprises one or more reagents selected from the group consisting of: a buffer, a culture medium, and a fructose-1,6-bisphosphate standard.

Item 9. A method for preparing the optical probe according to any one of Items 2 to 4, comprising: providing the host cell according to Item 7, culturing the host cell under conditions where the optical probe is expressed, and isolating the optical probe.

Item 10. Use of the optical probe according to any one of items 2 to 4, the nucleic acid sequence described in item 5, the nucleic acid construct according to item 6 or the host cell according to item 7 in detecting fructose-1,6-bisphosphate in a sample, screening compounds, and localizing fructose-1,6-bisphosphate inside or outside the cell,
preferably,
the detection of fructose-1,6-bisphosphate in a sample comprises the steps of: contacting the optical probe or host cell with the sample, determining an optical change of the optically active polypeptide, and detecting fructose-1,6-bisphosphate in the sample according to the optical change of the optically active polypeptide,
the screening of compounds comprises the steps of: contacting the optical probe or the host cell with a candidate compound in a system containing fructose-1,6-bisphosphate, determining an optical change of an optically active polypeptide, and screening the compound according to the optical change of the optically active polypeptide; preferably, the screening compound comprises the steps of: contacting the host cell with a candidate compound in a system containing fructose-1,6-bisphosphate, and the optical change of the optically active polypeptide indicates whether the candidate compound regulates the cell's uptake of fructose-1,6-bisphosphate,
the intracellular/extracellular localization of fructose-1,6-bisphosphate comprises the steps of contacting a system containing fructose-1,6-bisphosphate with the optical probe or the host cell, and detecting the optical change of the optically active polypeptide,
more preferably, the system is a solution system, a cell system or a subcellular system.

### Example

The fructose-1,6-bisphosphate optical probes provided by the invention are described in detail below in combination with the examples, but they cannot be understood as defining the scope of protection of the invention.

### I. Experimental materials and reagents

The conventional molecular biology cloning methods for genetic engineering and cell culture and imaging methods mainly used in examples are well known to those skilled in the art, for example, Roskams, J., Molecular Biology Laboratory Handbook; J. Sambrook, D. W. Russell ed, Molecular Cloning: A Laboratory Manual, translated by Peitang Huang et al. (3rd edition, August 2002, Science Press); R. I. Freshney et al., Culture of Animal Cells: a Manual of Basic Technique (5th edition), translated by Jingbo Zhang and Cunquan Xu; Juan S. Bonifacino, M. Daso, et al., Short Protocols in Cell Biology, translated by Jingbo Zhang et al.

The pCDF-cpYFP based, pCDF-fructose-1,6-bisphosphate-binding protein plasmid used in examples was constructed by the Protein Laboratory of East China University of Science and Technology, and the pCDF plasmid vector was purchased from Invitrogen. All the primers used for PCR were synthesized, purified, and identified to be correct by mass spectrometry (MS) by Shanghai Generay Biotech Co., Ltd and BGI Genomics. The expression plasmids constructed in examples were all sequenced by BGI Genomics and Jie Li Biology. For each example, Taq DNA polymerase was purchased from Dongsheng Bio, pfu DNA polymerase was purchased from Tiangen Biochemical Technology (Beijing) Co., Ltd, primeSTAR DNA polymerase was purchased from Takara, and all three polymerases were purchased with corresponding polymerase buffer and dNTPs. Restriction enzymes such as BamHI, Bglll, Hindlll, Ndel, Xhol, EcoRI, Spel, T4 ligase, and T4 phosphorylase (T4 PNK) were purchased from Fermentas with corresponding buffers and the like. Transfection reagent Lip2000 Kit was purchased from Invitrogen. Fructose-1,6-bisphosphate and other compounds were purchased from Sigma. Chemical reagents such as inorganic salts were purchased from Sigma-Aldrich unless specifically stated. HEPES salts, ampicillin (Amp) and puromycin were purchased from Ameresco. The 96-well detection blackboard and the 384-well fluorescence detection blackboard were purchased from Grenier Company.

The DNA purification kit used in examples was purchased from BBI (Canada), and the common Plasmid Mini Preparation Kit was purchased from Tiangen Biochemical Technology (Beijing) Co., Ltd. Clonal strain Mach1 was purchased from Invitrogen. Nickel column affinity chromatography columns and desalting column packing were from GE Healthcare.

The main instruments used in examples include: Biotek Synergy 2 multi-purpose microplate reader (Bio-Tek, USA), X-15R high-speed refrigerated centrifuge (Beckman, USA), Microfuge22R tabletop high-speed refrigerated centrifuge (Beckman, USA), PCR amplimer (Biometra, Germany), ultrasonic disruptor (Ningbo SCIENTZ), nucleic acid electrophoresis instrument (Shennengbocai company), spectrofluorometer (Varian, USA), CO₂ constant temperature cell culture incubator (SANYO), and inverted fluorescence microscope (Nikon, Japan).

### II. Molecular biology methods and cellular experimental methods

### 11.1 Polymerase chain reaction (PCR):

1. PCR amplification of target fragments:
   This method was mainly used for gene fragment amplification and colony PCR to identify positive clones. The reaction system for PCR amplification was as follows: template sequence 0.5-1 µl, the forward primer (25 µM) 0.5 µl, the reverse primer (25 µM) 0.5 µl, 10×pfu buffer 5 µl, pfu DNA polymerase 0.5 µl, dNTP (10 mM) 1 µl, sterilized ultrapure water (ddH2O) 41.5-42 µl, to a total volume of 50 µl. The PCR amplification program was as follows: denaturation at 95 °C for 2-10 min, 30 cycles (94-96 °C for 30-45 s, 50-65 °C for 30-45 s, 72 °C for a period (600 bp/min)), and extension at 72 °C for 10 min.
2. PCR amplification of long fragments (>2500bp):
   Long fragment amplification, primarily inverse PCR amplification vector, used in the invention is a technique used to obtain position directed mutations in examples below. Reverse PCR primers were designed at the variation position, where the 5 'end of one primer contained the variant nucleotide sequence. Amplified products contained the corresponding mutation position. The reaction system of amplification PCR of long fragments were as follows: template sequence (10 pg-1 ng) 1 µl, the forward primer (25 µM) 0.5 µl, the reverse primer (25 µM) 0.5 µl, 5×PrimerSTAR buffer 10 µl, PrimerSTAR DNA polymerase 0.5 µl, dNTPs (2.5 mM) 4 µl, sterilized ultrapure water (ddH2O) 33.5 µl, to a total volume of 50 µl. The PCR amplification program was as follows: denaturation at 95 °C for 5 min, 30 cycles (98 °C for 10 s, 50-68 °C for 5-15 s, 72 °C for a period (1000 bp/min)), and extension at 72 °C for 10 min; or denaturation at 95 °C for 5 min, 30 cycles (98 °C for 10 s, 68 °C for a period (1000 bp/min)), and extension at 72 °C for 10 min.

### 11.2 Endonuclease digestion reaction:

The system for double digestion of plasmid vectors was as follows: plasmid vector 20 µl (approximately 1.5 µg), 10×Buffer 5 µl, restriction endonuclease 11-2 µl, restriction endonuclease 21-2 µl, made up to a total volume of 50 µl with sterilized ultrapure water.

Reaction condition was 37°C, 1-7 h.

### II.3 5' phosphorylation reaction of DNA fragments

The ends of plasmids or genomes extracted from microorganisms contain phosphate groups, but PCR products do not contain. Therefore, it is necessary to perform phosphate group addition reaction on the 5'-end bases of PCR products, only DNA molecules containing phosphate groups at the ends can occur ligation reaction. The phosphorylation reaction system was as follows: PCR product fragment DNA sequence 5-8 µL, 10×T4 polynucleotide buffer 1 µL, T4 polynucleotide (T4 µL 1 µL), phosphorylation reaction ligation ultrapure water 0-3 µL, total volume 10 µL. The reaction conditions were 37°C, and inactivation at 72°C for 20 min after reacting for 30 min to 2 h.

### II.4 Ligation of target fragments and vectors

There are differences in the ligation method between different fragments and vectors, and three ligation methods were used in this invention.
1. Blunt-end ligation of blunt-ended short fragments and linearized vectors
   The principle of this method is that the blunt-end product obtained by PCR is phosphorylated at the 5' end of the DNA fragment under the action of T4 PNK, and then connected with the linearized vector under the action of PEG4000 and T4 DNA ligase to obtain a recombinant plasmid. The homologous recombination connection system was as follows: DNA fragment treated with T4 PNK 4µL, linearized vector fragment 4µL, PEG4000 1µL, 10×T4 ligase buffer 1µL, T4 DNA ligase 1µL, a total of 10µL. The reaction conditions was 22°C, 30 min.
2. Ligation of DNA fragments containing sticky ends and vector fragments containing sticky ends
   DNA fragments cut by restriction endonucleases usually produce sticky ends that stick out, so they can be ligated with vector fragments containing complementary sticky ends to form recombinant plasmids. The ligation reaction system is as follows: PCR product fragment DNA after digestion 1-7 µL, plasmid after digestion 0.5-7 µL , 10×T4 ligase buffer 1 µL, T4 DNA ligase 1 µL, and sterilized ultrapure water to make up the total volume 10 µL. Reaction condition was 16°C, 4-8 h.
3. The self-circularization ligation reaction of the 5' phosphorylated DNA fragment product after position-directed mutation introduced by inverse PCR
   The 5'-end phosphorylated DNA fragment was ligated to the 3'-end and 5'-end of the linearized vector through self-circularization ligation reaction to obtain a recombinant plasmid. The self-cyclization ligation reaction system is as follows: phosphorylation reaction system 10 µL, T4 ligase (5 U/µL) 0.5 µL, total volume 10.5 µL. Reaction condition was 16°C, 4-16 h.

### 11.5 Preparation and transformation of competent cells

### Preparation of competent cells:

1. A single colony (such as Mach1) was picked and inoculated into 5 mL LB medium and shake it at 37°C overnight.
2. 0.5-1 mL of overnight culture was transfered into 50 mL LB medium and cultured at 37°C, 220 rpm for 3 to 5 h until OD600 reached 0.5.
3. The cells were precooled in ice bath for 2 h.
4. 4 °C, centrifugation at 4000 rpm for 10 min.
5. The supernatant was discarded, the cells were resuspended with 5 ml of precooled buffer, and resuspension buffer was added again after homogenization to a final volume of 50 ml.
6. The bacteria was bathed in ice for 45 min.
7. The bacteria was centrifuged at 4000 rpm for 10 min at 4 °C, and resuspended with 5 ml ice precooled storage buffer.
8. 100 µl of broth was loaded to each EP tube and frozen at -80 °C or in liquid nitrogen.

Resuspension buffer: CaCl₂ (100 mM), MgCl₂ (70 mM), NaAc (40 mM)
Transformation of competent cells: 0.5 mL DMSO, 1.9 mL 80% glycol, 1 mL 10×CaCl₂ (1 M), 1 mL 10×MgCl₂ (700 mM), 1mL 10×NaAc (400 mM), 4.6 mL ddH₂O

### Transformation of compenent cells:

1. 100 µl competent cells were taken and thawed on an ice bath.
2. The appropriate volume of ligation product was added, mixed by gently pipetting, and incubated in ice bath for 30 min. The volume of ligation product normally added was less than 1/10 of the volume of competent cells.
3. The broth was put into a 42 °C water bath for heat shock for 90 s and quickly transferred to an ice bath for 5 min.
4. 500 µl LB was added and incubated at 200 rpm on a constant temperature shaker at 37 °C for 1 hour.
5. The broth was centrifuged at 4000 rpm for 3 min and 200 µl supernatant was added to mix the bacteria well and spread evenly on the surface of the agar plate containing appropriate antibiotics, and the plate is inverted in a 37 °C incubator overnight.

### II.6 Expression, purification, and fluorescent detection of proteins

1. An expression vector (such as a pCDF-based fructose-1,6-bisphosphate optical probe expression vector) was transform into BL21 (DE3) cells, and invert- cultured overnight, and clones were picked from the plate into a 250 ml Erlenmeyer flask, and placed in a 37°C shaker, cultured at 220 rpm to OD=0.4-0.8, add 1/1000 (v/v) IPTG (1 M), and induced expression at 18 °C for 24-36 h.
2. After induction, cells were harvested by centrifugation at 4000 rpm for 30 min, resuspended in 50 mM phosphate buffer and disrupted ultrasonically until the broth were clear, centrifuged at 9600 rpm at 4 °C for 20 min.
3. The centrifuged supernatant was purified by a self-packed nickel-column affinity chromatography column to obtain the protein, and the protein after nickel-column affinity chromatography was then passed through a self-packed desalting column to obtain the protein dissolved in 100 mM HEPES buffer (pH 7.4).
4. After the purified protein was identified by SDS-PAGE, the probe was diluted with assay buffer (100 mM HEPES, 100 mM NaCl, pH 7.4) into a protein solution with a final concentration of 0.2-5 µM. Fructose-1,6-bisphosphate was prepared as a stock solution at a final concentration of 50 mM with assay buffer (100 mM HEPES, 100 mM NaCl, pH 7.4).
5. 100 µl of 1 µM protein solution was taken and incubated at 37 °C for 10 min,tryptophan for titration was added, and measured the fluorescence intensity of the protein at 528 nm after excitation at 420 nm and fluorescence intensity of the protein at 528 nm after excitation at 485 nm. The fluorescence excitation and emission measurement of the samples were completed by a multifunctional fluorescence microplate reader.
6.100 µl of 1 µM protein solution was taken, incubated at 37 °C for 10 min, tryptophan was added, and measured the absorption and fluorescence spectra of the protein. The determination of the absorption spectrum and fluorescence spectrum of the sample was completed by a spectrophotometer and a fluorescence spectrophotometer.

### 11.6 Expression, purification, and fluorescent detection of proteins

1. The pCDNA3.1+-based fructose-1,6-bisphosphate optical probe plasmid was transfected into HEK293 cells by the transfection reagent Lipofectamine2000 (Invitrogen), and cultured in a cell incubator at 37°C, 5% CO₂. Fluorescence detection was performed 24-36 h after the exogenous gene was fully expressed.
2. After induction of expression, adherent HEK293 cells were washed three times with PBS and placed in HBSS solution for fluorescence microscopy and microplate reader assays, respectively.

### Example 1. Fructose-1,6-bisphosphate binding protein plasmid

The CggR truncated gene in the Bacillus subtilis gene was amplified by PCR, and the PCR product was recovered after gel electrophoresis and digested with Hindlll and Xhol, the pCDF vector was double-digested at the same time. After ligation with T4 DNA ligase, the product was used to transform DH5α. The transformed DH5α was coated on an LB plate (streptomycin 100ug/mL) and cultured at 37°C overnight. The grown DH5α transformants were subjected to plasmid extraction and PCR identification. Positive plasmids were sequenced to be correct for subsequent plasmid construction.

### Example 2: Expression and detection of optical probes with cpYFP inserted at different positions

In this example, based on pCDF-CggR (88-340), the following positions were selected for insertion of cpYFP according to the crystal structure of fructose-1,6-bisphosphate binding protein to obtain the corresponding pCDF-CggR (88-340)-cpYFP plasmids: 174/175, 174/176, 174/177, 174/178, 174/179, 174/180, 174/181, 174/182, 174/183, 174/184, 174/185, 155/176, 175/177, 175/178, 175/179, 175/180, 175/181, 175/182, 175/183, 175/184, 175/185, 176/177, 176/178, 176/179, 176/180, 176/181, 176/182, 176/183, 176/184, 176/185, 177/178, 177/179, 177/180, 177/181, 177/182, 177/183, 177/184, 177/185, 178/179, 178/180, 178/181, 178/182, 178/183, 178/184, 178/185, 179/180, 179/181, 179/182, 179/183, 179/184, 179/185, 180/181, 180/182, 180/183, 180/184, 180/185, 181/182, 181/182, 181/183, 181/184, 181/185, 182/183, 182/184, 182/185, 183/184, 183/185, 184/185, 201/202, 201/203, 201/204, 201/205, 201/206, 201/207, 201/208, 202/203, 202/204, 202/205, 202/206, 202/207, 202/208, 203/204, 203/205, 203/206, 203/207, 203/208, 204/205, 204/206, 204/207, 204/208, 205/206, 205/206, 205/207, 205/208, 206/207, 206/208 and/or 207/208.

The DNA fragment of cpYFP was generated by PCR, and the cpYFP terminal homologous sequence was introduced through the 5' end of the primer, and the pCDF-fructose-1,6-bisphosphate binding protein linearized vector was generated by PCR amplificationand and its 5' and 3' ends respectively carried completely consistent sequences (15bp~20bp) corresponding to the two ends of cpYFP. The linearized pCDF-CggR (88-340) and cpYFP fragments were subjected to homologous recombination under the action of Hieff Clone Enzyme. The product was transformed into DH5α, and the transformed DH5α was spread on LB plate (streptomycin 100ug/mL) and cultured at 37°C overnight. The positive clones identified by PCR were sequenced after extraction of plasmids. Sequencing was performed by JieLi Biology.

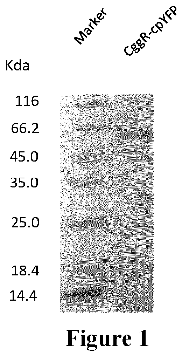

After being sequenced correctly, the recombinant plasmid was transformed into BL21 (DE3) to induce expression, and the protein was purified, and the size was around 57 Kda by SDS-PAGE electrophoresis. This size is consistent with the size of the CggR (88-340) -cpYFP fusion protein containing the His-tag purification tag expressed by pCDF-CggR (88-340) -cpYFP. The results are shown in Figure 1.

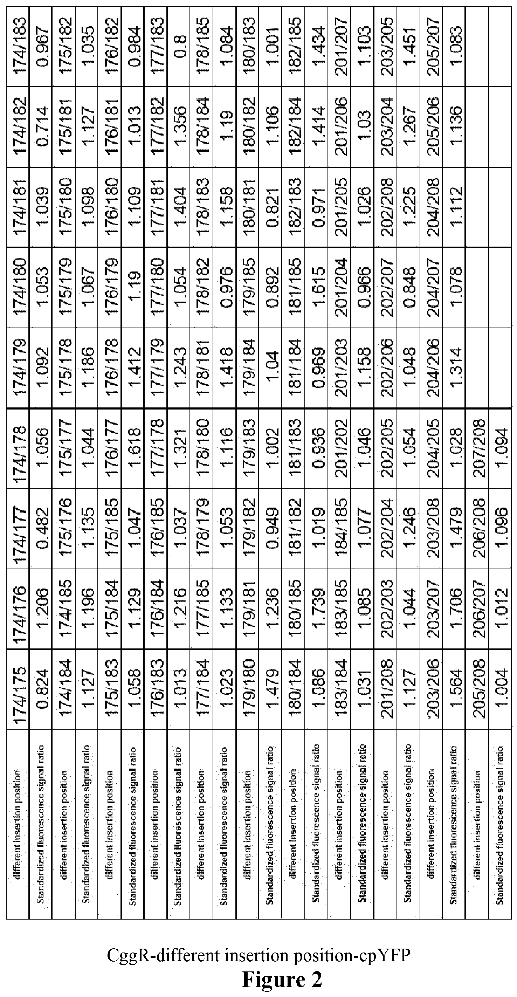

The supernatant of E. coli expressing CggR(88-340)-cpYFP fusion protein was used for fructose-1,6-bisphosphate response screening, and the detection signal of the fusion fluorescent protein containing 1mM fructose-1,6-bisphosphate was divided by the detection signal of the fusion fluorescent protein without fructose-1,6-bisphosphate. The results are shown in Figure 2. The detection results show that the optical probes with a response to fructose-1,6-bisphosphate of more than 1.4 times are inserted at 176/177, 176/178, 177/181, 179/180, 182/184, 182/185, 203/206, 203/207 and 203/208 or the corresponding amino acid positions of their family proteins. The screening results are shown in the Table below.

### Example 3. Expression and detection of cpGFP optical probes with different insertion positions

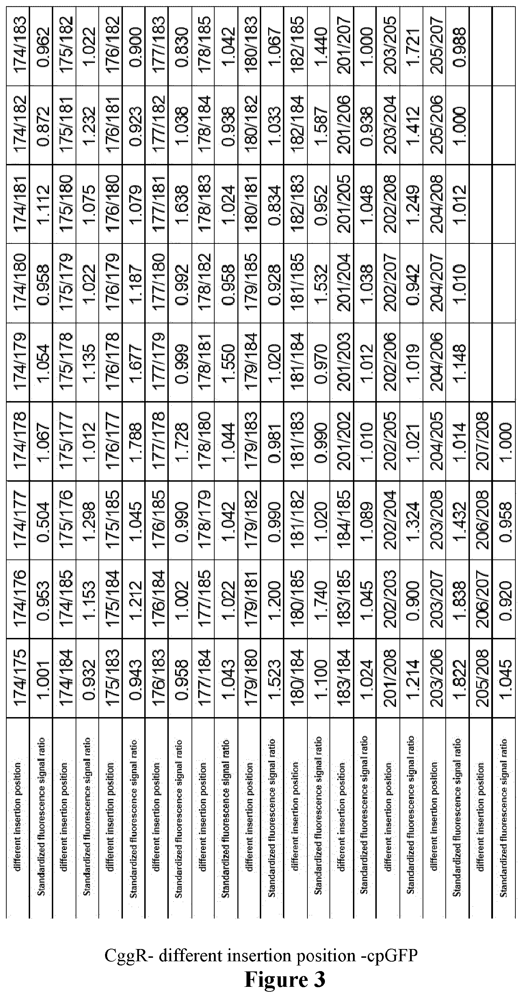

According to the method in Example 2, cpYFP was replaced with cpGFP to construct a fructose-1,6-bisphosphate green fluorescent protein fluorescent probe. As shown in Figure 3, the detection results show that the optical probes that respond to fructose-1,6-bisphosphate more than 1.4 times include optical probes inserted at positions 176/177, 176/178, 177/181, 182/184, 182/185, 203/204, 203/205, 203/206, 203/207 and 203/208 or the corresponding amino acid positions of their family proteins.

### Example 4. Expression and detection of optical probes having cpBFP inserted at different positions

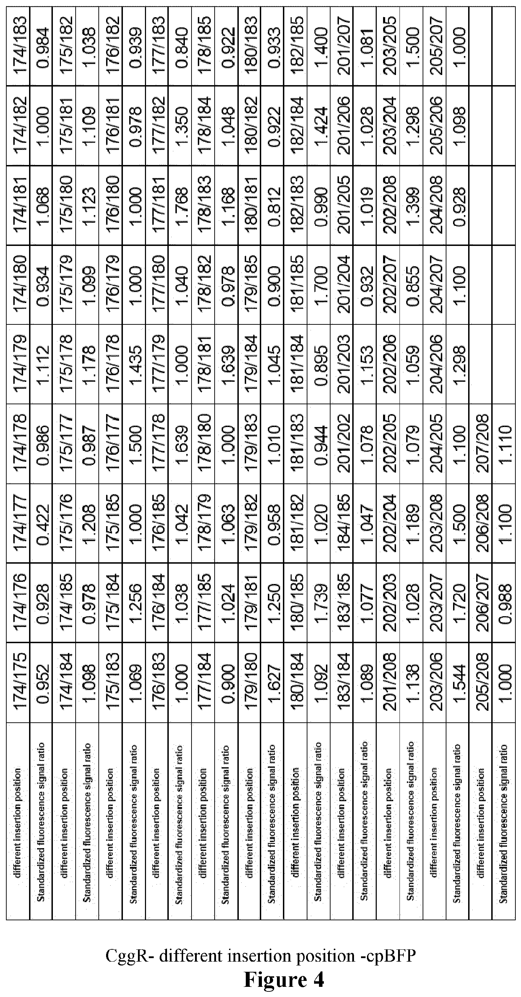

According to the method in Example 2, cpYFP was replaced with cpBFP to construct a fructose-1,6-bisphosphate blue fluorescent protein fluorescent probe. As shown in Figure 4, the detection results show that the optical probes that respond to fructose-1,6-bisphosphate more than 1.5 times are inserted at 176/177, 176/178, 177/178, 177/181, 178/181, 182/184, 182/185, 203/205, 203/206, 203/207 and 203/208 positions or the corresponding amino acid positions of their family proteins.

### Example 5, Expression and detection of linker-mutated CPYFP optical probes

Optical probe mutants were constructed based on PCDF-CggR(88-340)-176/177-CPYFP, PCDF-CggR(88-340)-176/178-CPYFP, PCDF-CggR(88-340)-177/181-CPYFP, PCDF-CggR(88-340)-179/180-CPYFP, PCDF-CggR(88-340)-182/184-CPYFP, PCDF-CggR(88-340)-182/185-CPYFP, PCDF-CggR(88-340)-203/206-CPYFP, PCDF-CggR(88-340)-203/207-CPYFP and PCDF-CggR-203/208(88-340)-CPYFP. Linearize the plasmid PCDF-CggR(88-340)-176/177-CPYFP, PCDF-CggR(88-340)-176/178-CPYFP, PCDF-CggR(88-340)-177/181-CPYFP, PCDF-CggR(88-340)-178/180-CPYFP, PCDF-CggR(88-340)-182/184-CPY by PCR. The linkers of primers for plasmid were set to NNK random mutation, and the obtained PCR products were phosphorylated under the action of PNK, T4 DNA ligase and PEG4000, and then transformed and constructed, and tested, and mutants with a response to fructose-1,6-bisphosphate greater than 2 times were screened and sequenced by Jieli Biology. The screened mutants are shown in the Table below:

### Linker mutant

| Serial numb er | Insertion position | Mutation position | Standardized fluorescence signal ratio |
|---|---|---|---|
| 1 | 176/177 | CggR:G177A /L178Y | 3.03 |
| 2 | 176/177 | CggR:G177I /L178F | 2.92 |
| 3 | 176/177 | CggR:G177L /L178Y | 3.44 |
| 4 | 176/177 | CggR:G177M/L178N | 4.54 |
| 5 | 176/177 | CggR:G177N/L178Y | 2.85 |
| 6 | 176/177 | CggR:G177M/L178N/Q185S | 3.22 |
| 7 | 176/177 | CggR:G177M/L178N/Q185A | 4 |
| 8 | 176/177 | CggR:G177M/L178N/Q185T | 4 |
| 9 | 176/177 | CggR:G177M/L178N/0185D | 4.17 |
| 10 | 176/177 | CggR:G177M/L178N/T148S | 3.22 |
| 11 | 176/177 | CggR:G177M/L178N/F271Y | 3.7 |
| 12 | 177/181 | CggR:D181F/V182F | 3.56 |
| 13 | 177/181 | CggR:D181L/V182L | 3.33 |
| 14 | 179/180 | CggR:E180G/D181G | 1.68 |
| 15 | 182/184 | CggR:N184V/Q185P | 4.93 |
| 16 | 182/184 | CggR:N184V/Q185P/D181E/V182G/G177L CPYFP:Y1V | 11.6 |
| 17 | 182/184 | CggR:N184V/Q185P/D181E/V182G/G177L CPYFP:Y1I | 12 |
| 18 | 182/184 | CggR:N184V/Q185P/G177L CPYFP:Y1V | 7.4 |
| 19 | 182/184 | CggR:N184V/Q185P/G177L CPYFP:Y1I | 5.5 |
| 20 | 182/184 | CggR:N184V/Q185P/D181E/V182G CPYFP:Y1V | 9.6 |

### Example 6. Performance of Optical probe and its mutants

Exemplarily, the three purified fructose-1,6-bisphosphate optical probes numbered 4, 15, and 17 in Example 5 were treated with 0 mM and 5 mM fructose-1,6-bisphosphate for 10 min, respectively, and then the fluorescence spectra were detected using a fluorescence spectrophotometer.

Determination of excitation spectra: Excitation spectra were recorded with an excitation range of 370 nm to 510 nm and an emission wavelength of 530 nm, with readings every 5 nm. The results shows that the probes have two excitation peaks at about 410 and 490nm.

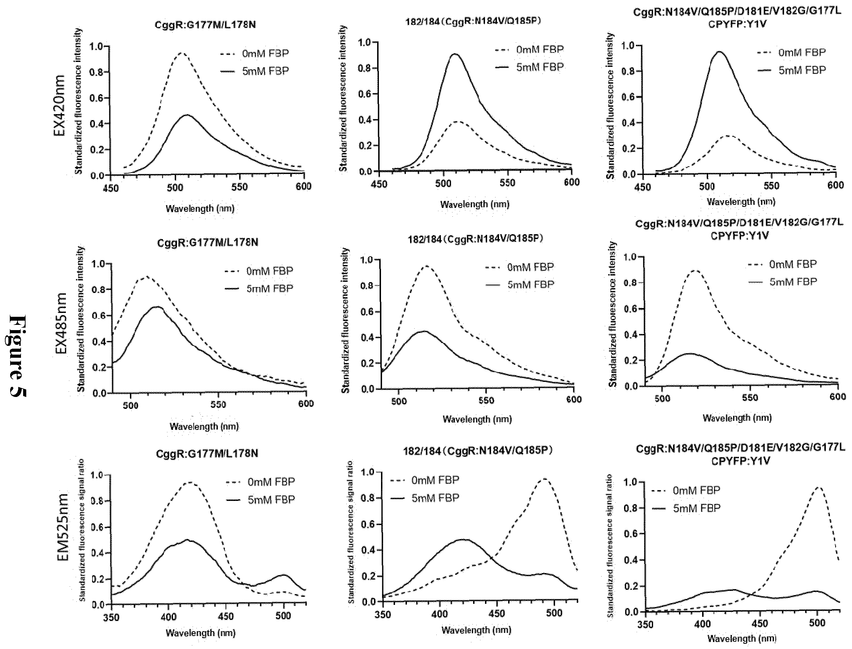

Determination of emission spectra: The excitation wavelengths were fixed at 420 nm and 460 nm, respectively, and the emission spectra at 470-600 nm and 490-600 nm were recorded and read every 5 nm. The excitation and emission spectra are shown in Figure 5.

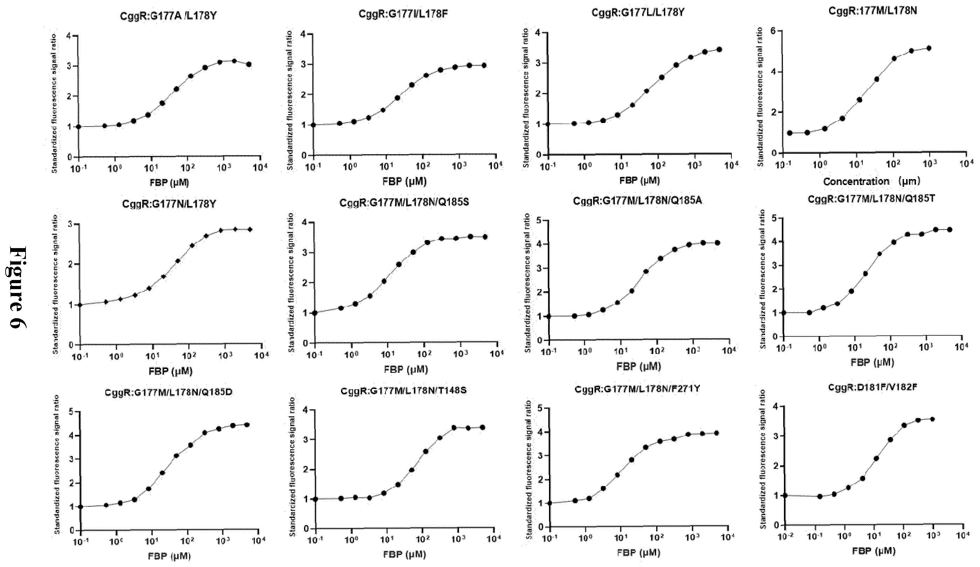

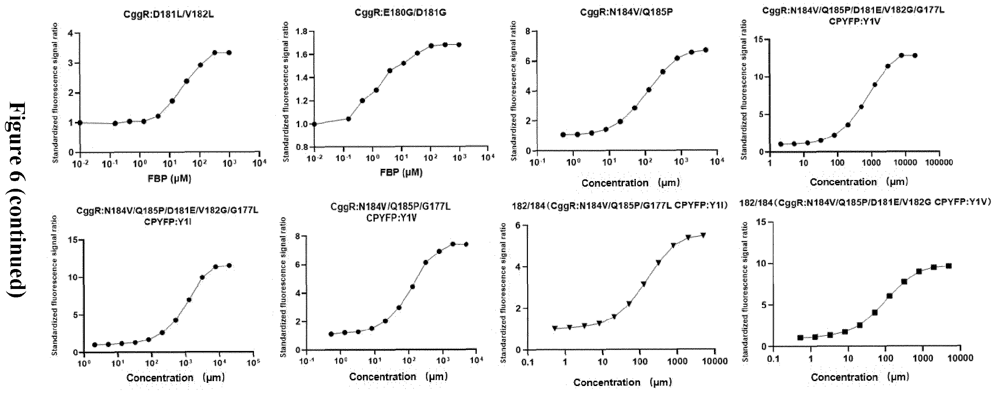

The twenty purified fructose-1,6-bisphosphate optical probes numbered 1-20 in the Example 5 were subjected to fructose-1,6-bisphosphate detection at a concentration gradient (0-5 mM). After the purified probes were treated for 10 min, the changes of the ratio in the fluorescence intensity at 528 nm emission under 420 nm excitation to the fluorescence intensity at 528 nm emission under 485 nm excitation were detected. The results are shown in Figure 6, and the Kd (binding constant) of the twenty fructose-1,6-bisphosphate optical probes are 37 µM, 25 µM, 73 µM, 11 µM, 38 µM, 12 µM, 36 µM, 22 µM, 32 µM, 74 µM, 12 µM, 5.7 µM, 6.2 µM, 3.3 µM, 360 µM, 240 µM, 769 µM, 1524 µM, 117 µM, 148 µM and 92 µM, respectively.

The twenty purified fructose-1,6-bisphosphate optical probes numbered 1-20 in the Example 5 were subjected to reactivity detection with eight similar substrates.

### Example 7. Suborganelle localization and performance within suborganelles of optical probes

In this example, different localization signal peptides were used to fuse the optical probe 182/184 (CggR: N184V/Q185P/D181E/V182G CPYFP: Y1V No. 20 in Example 5) to localize the optical probe to different organelles.

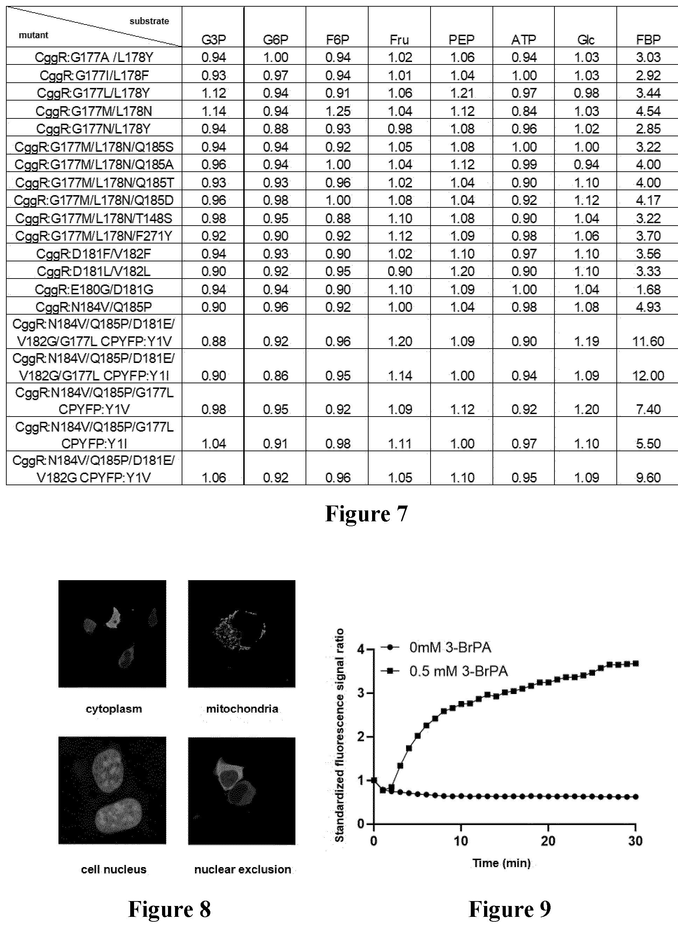

HeLa cells were transfected with optical probe plasmids fused with different localization signal peptides for 36 h, rinsed with PBS, placed in HBSS solution, and subjected to fluorescence detection under the FITC channel under an inverted fluorescence microscope. The results are shown in Figure 8. The fructose-1,6-bisphosphate optical probe can be localized to subcellular organelles including cytoplasm, nucleus, mitochondria, and nuclear exclusion by fusing with different specific localization signal peptides. Fluorescence was shown in different subcellular structures, and the distribution and intensity of fluorescence varied.

After H1299 cells were transfected with the cytoplasm-expressed optical probe plasmid for 36 h, they were washed with PBS and placed in HBSS solutions containing 0 mM 3-BrPA and 0.5 mM 3-BrPA, and the changes in the ratio of the fluorescence intensity at 528 nm emission and at 420 nm excitation and the fluorescence intensity at 528 nm emission at 485 nm excitation were detected over a 30-min period. The results are shown in Figure 9. The ratio of 420/485 of the sample with 3-BrPA added gradually increased, reaching up to 3.7 times the initial value, while the 420/485 of the control group without 3-BrPA added remained unchanged at 0.63.

### Example 8. Optical Probe-Based High-Throughput Compound Screening in Living Cells

In this example, we used HeLa cells expressing 182/184 (CggR: N184V/Q185P/D181E/V182GCPYFP: Y1V) in the cytoplasm for high-throughput compound screening.

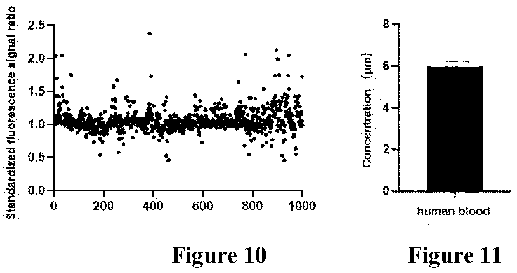

The transfected HeLa cells were rinsed with PBS, placed in HBSS solution (without fructose-1,6-bisphosphate) for 1 hour, and then treated with 10µM compounds for 1 hour. Various compounds were added to each sample. Use a microplate reader to record the ratio of the fluorescence intensity at 420 nm excitation at 528 nm emission to the fluorescence intensity at 485 nm excitation at 528 nm emission. Normalization was performed with samples not treated with any compound as controls. The results are shown in Figure 10. Among the 1000 compounds used, most of the compounds had little effect on the concentration of fructose-1,6-bisphosphate in cells. 38 compounds can increase the concentration of fructose-1,6-bisphosphate in cells, and 13 compounds can significantly reduce the concentration of fructose-1,6-bisphosphate in cells.

### Example 9. Quantitative Detection of fructose-1,6-bisphosphate in blood with optical probes

In this example, fructose-1,6-bisphosphate in the blood supernatants of mice and humans was analyzed using purified 179/180 (CggR: E180G/D181G) with a Kd of approximately 1 µM.

After 179/180 (CggR: E180G/D181G) was mixed with diluted blood supernatant for 10 min, the ratio of the fluorescence intensity the fluorescence intensity at 420nm excitation and 528nm emission to the fluorescence intensity the fluorescence intensity at 485nm excitation and 528nm were detected using an ELISA detector. The results are shown in Figure 11, the fructose-1,6-bisphosphate content in the blood of healthy people is about 6µM.

It can be seen from the above examples that the fructose-1,6-bisphosphate optical probe provided by the present invention has relatively small protein molecular weight and is easy to mature, with large dynamic changes in fluorescence and good specificity, and it can be expressed in cells by genetic manipulation, and can locate and quantitatively detect tryptophan in and out of cells in real time; and it can perform high-throughput compound screening.

### OTHER EMBODIMENTS

This specification describes a number of embodiments. It should be understood, however, that various modifications that those skilled in the art will come to know from reading this specification without departing from the spirit and scope of the invention should also be included within the scope of the appended claims.

### sequences of the present invention

1>MNQLIQAQKKLLPDLLVVMQKRFEILQYIRLTEPIGRRSLSASLGISERVLRGEVQFLKEQNLVEIK
TNGMTLTEEGYSLLSILEDTMKDVLGLTVLEKTLKERLNLRDAIIVSGDSDQSPWVKKELGRAAVAC
MKKRFSGKNIVAVTGGTTIEAVAEMMTPDSKNRELLFVPARGGLGENVKNQANTICAHMAEKASG
TYRLLFVPGQLSQGAYSSIIEEPSVKEVLTTIKSASMLVHGIGEAKTMAERRNTPLEDLKKIDENHAV
TEAFGYYFNSDGEVVHKVHSVGMQLDDLDAIPDIIAVAGGSSKAGAIEAYFKKPRNTVLVTDEGAA
KKLLRDE

2>YNSDNVYIMADKQKNGIKTNFKIRHNVEDGSVQLADHYQQNTPIGDGPVLLPDNHYLSFQSVLS
KDPNEKRDHMVLLEFVTAAGITLGMDELYNVDGGSGGTGSKGEELFTGVVPILVELDGDVNGHKF
SVSGEGEGDATYGKLTLKLICTTGKLPVPWPTLVTTLGYGLKCFARYPDHMKQHDFFKSAMPEGY
VQERTIFFKDDGNYKTRAEVKFEGDTLVNRIELKGIDFKEDGNILGHKLEYN

3>VSERMYPEDGVLKSEIKKGLRLKDGGHYAAEVKTTYKAKKPVQLPGAYIVDIKLDIVSHNEDYTIV
EQCERAEGRHPTGGRDELYKGGTGGSLVSKGEEDNMAIIKEFMRFKVHMEGSVNGHEFEIEGEG
EGRPYEAFQTAKLKVTKGGPLPFAWDILSPQFTYGSKAYIKHPADIPDYFKLSFPEGFRWERVMNF
EDGGIIHVNQDSSLQDGVFIYKVKLRGTNFPPDGPVMQKKTMGWEA

4>MGGRSKKPAKNLKMPGVYYVDRRLERIKEADKETYVEQHEVAVARYCDLPSKLGHKLNGGTG
GSMVSKGEELIKENMHMKLYMEGTVNNHHFKCTSEGEGKPYEGTQTMRIKVVEGGPLPFAFDILA
TSFMYGSKTFINHTQGIPDFFKQSFPEGFTWERVTTYEDGGVLTATQDTSLQDGCLIYNVKIRGVN
FPSNGPVMQKKTLGWEASTEMLYPADGGLEGRSDMALKLVGGGHLICNLKTTYRSKK

5>MVSKGEEDNMAIIKEFMRFKVHMEGSVNGHEFEIEGEGEGRPYEGTQTAKLKVTKGGPLPFAW
DILSPQFMYGSKAYVKHPADIPDYLKLSFPEGFKWERVMNFEDGGVVTVTQDSSLQDGEFIYKVKL
RGTNFPSDGPVMQKKTMGWEASSERMYPEDGALKGEIKQRLKLKDGGHYDAEVKTTYKAKKPV
QLPGAYNVNIKLDITSHNEDYTIVEQYERAEGRHSTGGMDELYK

6>NVYIKADKQKNGIKANFKIRHNIEDGGVQLAYHYQQNTPIGDGPVLLPDNHYLSVQSILSKDPNE
KRDHMVLLEFVTAAGITLGMDELYKGGTGGSMVSKGEELFTGVVPIQVELDGDVNGHKFSVSGEG
EGDATYGKLTLKFICTTGKLPVPWPTLVTTLTYGVQCFSRYPDHMKQHDFFKSAMPEGYIQERTIF
FKDDGNYKTRAEVKFEGDTLVNRIELKGIDFKEDGNILGHKLEYN

7>NVYIKADKQKNGIKANFKIRHNIEGGGVQLAYHYQQNTPIGDGPVLLPDNHYLSVQSILSKDPNE
KRDHMVLLEFVTAAGITLGMDELYKGGTGGSESMVSKGEELFTGVVPIQVELDGDVNGHKFSVSG
EGEGDATYGKLTLKFICTTGKLPVPWPTLVTTLSHGVQCFSRYPDHMKQHDFFKSAMPGGYIQER
TIFFKDDGNYKTRAEVKFEGDTLVNRIELKGIDFKEDGNILGHKLEYN

8>MSELITENMHMKLYMEGTVNNHHFKCTSEGEGKPYEGTQTMRIKVVEGGPLPFAFDILATSFMY
GSKTFINHTQGIPDFFKQSFPEGFTWERVTTYEDGGVLTATQDTSLQDGCLIYNVKIRGVNFPSNG
PVMQKKTLGWEASTEMLYPADGGLEGRADMALKLVGGGHLICNLKTTYRSKKPAKNLKMPGVYY
VDRRLERIKEADKETYVEQHEVAVARYCDLPSKLGHKLN

9>VSERMYPEDGALKSEIKKGLRLKDGGHYAAEVKTTYKAKKPVQLPGAYIVDIKLDIVSHNEDYTIV
EQCERAEGRHSTGGMDELYKGGTGGSLVSKGEEDNMAIIKEFMRFKVHMEGSVNGHEFEIEGEG
EGRPYEAFQTAKLKVTKGGPLPFAWDILSPQFMYGSKAYIKHPADIPDYFKLSFPEGFRWERVMN
FEDGGIIHVNQDSSLQDGVFIYKVKLRGTNFPPDGPVMQKKTMGWEA

10>SNAKDVLGLTLLEKTLKERLNLKDAIIVSGDSDQSPWVKKEMGRAAVACMKKRFSGKNIVAVT
GGTTIEAVAEMMTPDSKNRELLFVPARGYNSDNVYIMADKQKNGIKTNFKIRHNVEDGSVQLADH
YQQNTPIGDGPVLLPDNHYLSFQSVLSKDPNEKRDHMVLLEFVTAAGITLGMDELYNVDGGSGGT
GSKGEELFTGVVPILVELDGDVNGHKFSVSGEGEGDATYGKLTLKLICTTGKLPVPWPTLVTTLGY
GLKCFARYPDHMKQHDFFKSAMPEGYVQERTIFFKDDGNYKTRAEVKFEGDTLVNRIELKGIDFKE
DGNILGHKLEYNMNGEDVKNQANTICAHMAEKASGTYRLLFVPGQLSQGAYSSIIEEPSVKEVLNTI
KSASMLVHGIGEAKTMAQRRNTPLEDLKKIDDNDAVTEAFGYYFNADGEVVHKVHSVGMQLDDID
AIPDIIAVAGGSSKAEAIEAYFKKPRNTVLVTDEGAAKKLLRDE

11>SNAKDVLGLTLLEKTLKERLNLKDAIIVSGDSDQSPWVKKEMGRAAVACMKKRFSGKNIVAVT
GGTTIEAVAEMMTPDSKNRELLFVPARGYNSDNVYIMADKQKNGIKTNFKIRHNVEDGSVQLADH
YQQNTPIGDGPVLLPDNHYLSFQSVLSKDPNEKRDHMVLLEFVTAAGITLGMDELYNVDGGSGGT
GSKGEELFTGVVPILVELDGDVNGHKFSVSGEGEGDATYGKLTLKLICTTGKLPVPWPTLVTTLGY

GLKCFARYPDHMKQHDFFKSAMPEGYVQERTIFFKDDGNYKTRAEVKFEGDTLVNRIELKGIDFKE
DGNILGHKLEYNMNGEDVKNAANTICAHMAEKASGTYRLLFVPGQLSQGAYSSIIEEPSVKEVLNTI
KSASMLVHGIGEAKTMAQRRNTPLEDLKKIDDNDAVTEAFGYYFNADGEVVHKVHSVGMQLDDID
AIPDIIAVAGGSSKAEAIEAYFKKPRNTVLVTDEGAAKKLLRDE

12>SNAKDVLGLTLLEKTLKERLNLKDAIIVSGDSDQSPWVKKEMGRAAVACMKKRFSGKNIVAVT
GGTTIEAVAEMMTPDSKNRELLFVPARGYNSDNVYIMADKQKNGIKTNFKIRHNVEDGSVQLADH
YQQNTPIGDGPVLLPDNHYLSFQSVLSKDPNEKRDHMVLLEFVTAAGITLGMDELYNVDGGSGGT
GSKGEELFTGVVPILVELDGDVNGHKFSVSGEGEGDATYGKLTLKLICTTGKLPVPWPTLVTTLGY
GLKCFARYPDHMKQHDFFKSAMPEGYVQERTIFFKDDGNYKTRAEVKFEGDTLVNRIELKGIDFKE
DGNILGHKLEYNMNGEDVKNTANTICAHMAEKASGTYRLLFVPGQLSQGAYSSIIEEPSVKEVLNTI
KSASMLVHGIGEAKTMAQRRNTPLEDLKKIDDNDAVTEAFGYYFNADGEVVHKVHSVGMQLDDID
AIPDIIAVAGGSSKAEAIEAYFKKPRNTVLVTDEGAAKKLLRDE

13>SNAKDVLGLTLLEKTLKERLNLKDAIIVSGDSDQSPWVKKEMGRAAVACMKKRFSGKNIVAVT
GGTTIEAVAEMMTPDSKNRELLFVPARGYNSDNVYIMADKQKNGIKTNFKIRHNVEDGSVQLADH
YQQNTPIGDGPVLLPDNHYLSFQSVLSKDPNEKRDHMVLLEFVTAAGITLGMDELYNVDGGSGGT
GSKGEELFTGVVPILVELDGDVNGHKFSVSGEGEGDATYGKLTLKLICTTGKLPVPWPTLVTTLGY
GLKCFARYPDHMKQHDFFKSAMPEGYVQERTIFFKDDGNYKTRAEVKFEGDTLVNRIELKGIDFKE
DGNILGHKLEYNMNGEDVKNDANTICAHMAEKASGTYRLLFVPGQLSQGAYSSIIEEPSVKEVLNTI
KSASMLVHGIGEAKTMAQRRNTPLEDLKKIDDNDAVTEAFGYYFNADGEVVHKVHSVGMQLDDID
AIPDIIAVAGGSSKAEAIEAYFKKPRNTVLVTDEGAAKKLLRDE

14>SNAKDVLGTLLEKTLKERLNLKDAIIVSGDSDQSPWVKKEMGRAAVACMKKRFSGKNIVAVT
GGTTIEAVAEMMTPDSKNRELLFVPARGLLGEEGVNSDNVYIMADKQKNGIKTNFKIRHNVEDGSV
QLADHYQQNTPIGDGPVLLPDNHYLSFQSVLSKDPNEKRDHMVLLEFVTAAGITLGMDELYNVDG
GSGGTGSKGEELFTGVVPILVELDGDVNGHKFSVSGEGEGDATYGKLTLKLICTTGKLPVPWPTLV
TTLGYGLKCFARYPDHMKQHDFFKSAMPEGYVQERTIFFKDDGNYKTRAEVKFEGDTLVNRIELK
GIDFKEDGNILGHKLEYNVPANTICAHMAEKASGTYRLLFVPGQLSQGAYSSIIEEPSVKEVLNTIKS
ASMLVHGIGEAKTMAQRRNTPLEDLKKIDDNDAVTEAFGYYFNADGEVVHKVHSVGMQLDDIDAIP
DIIAVAGGSSKAEAIEAYFKKPRNTVLVTDEGAAKKLLRDE

15>SNAKDVLGLTLLEKTLKERLNLKDAIIVSGDSDQSPWVKKEMGRAAVACMKKRFSGKNIVAVT
GGTTIEAVAEMMTPDSKNRELLFVPARGLLGEEGINSDNVYIMADKQKNGIKTNFKIRHNVEDGSV
QLADHYQQNTPIGDGPVLLPDNHYLSFQSVLSKDPNEKRDHMVLLEFVTAAGITLGMDELYNVDG
GSGGTGSKGEELFTGVVPILVELDGDVNGHKFSVSGEGEGDATYGKLTLKLICTTGKLPVPWPTLV
TTLGYGLKCFARYPDHMKQHDFFKSAMPEGYVQERTIFFKDDGNYKTRAEVKFEGDTLVNRIELK
GIDFKEDGNILGHKLEYNVPANTICAHMAEKASGTYRLLFVPGQLSQGAYSSIIEEPSVKEVLNTIKS
ASMLVHGIGEAKTMAQRRNTPLEDLKKIDDNDAVTEAFGYYFNADGEVVHKVHSVGMQLDDIDAIP
DIIAVAGGSSKAEAIEAYFKKPRNTVLVTDEGAAKKLLRDE

16>SNAKDVLGLTLLEKTLKERLNLKDAIIVSGDSDQSPWVKKEMGRAAVACMKKRFSGKNIVAVT
GGTTIEAVAEMMTPDSKNRELLFVPARGGLGEEGVNSDNVYIMADKQKNGIKTNFKIRHNVEDGS
VQLADHYQQNTPIGDGPVLLPDNHYLSFQSVLSKDPNEKRDHMVLLEFVTAAGITLGMDELYNVD
GGSGGTGSKGEELFTGVVPILVELDGDVNGHKFSVSGEGEGDATYGKLTLKLICTTGKLPVPWPT
LVTTLGYGLKCFARYPDHMKQHDFFKSAMPEGYVQERTIFFKDDGNYKTRAEVKFEGDTLVNRIE
LKGIDFKEDGNILGHKLEYNVPANTICAHMAEKASGTYRLLFVPGQLSQGAYSSIIEEPSVKEVLNTI
KSASMLVHGIGEAKTMAQRRNTPLEDLKKIDDNDAVTEAFGYYFNADGEVVHKVHSVGMQLDDID
AIPDIIAVAGGSSKAEAIEAYFKKPRNTVLVTDEGAAKKLLRDE

17>tctaatgctaaagatgttctgggcctgaccctgctggagaagaccctgaaagaacgtctgaacctgaaagatgctatcattgtgagcg
gcgacagcgatcagagcccgtgggtgaagaaagagatgggtcgtgccgcggtggcgtgcatgaagaaacgcttcagcggcaagaac
atcgtggctgtgaccggcggtaccaccattgaggcggtggctgaaatgatgaccccggacagcaaaaaccgtgagctgctgtttgtgcca
gctcgcggctacaacagcgacaacgtctatatcatggccgacaagcagaagaacggcatcaaggccaacttcaagatccgccacaac
gtcgaggacggcagcgtgcagctcgccgaccactaccagcagaacacccccatcggcgacggccccgtgctgctgcccgacaacca
ctacctgagcttccagtccgtcctgagcaaagaccccaacgagaagcgcgatcacatggtcctgctggagttcgtgaccgccgccgggat
cactctcggcatggacgagctgtacaacgtggatggcggtagcggtggcaccggcagcaagggcgaggagctgttcaccggggtggtg
cccatcctggtcgagctggacggcgacgtaaacggccacaagttcagcgtgtccggcgagggcgagggcgatgccacctacggcaag
ctgaccctgaagctgatctgcaccaccggcaagctgcccgtgccctggcccaccctcgtgaccaccctcggctacggcctgaagtgcttc
gcccgctaccccgaccacatgaagcagcacgacttcttcaagtccgccatgcccgaaggctacgtccaggagcgcaccatcttcttcaag
gacgacggcaactacaagacccgcgccgaggtgaagttcgagggcgacaccctggtgaaccgcatcgagctgaagggcatcggcttc
aaggaggacggcaacatcctggggcacaagctggagtacaacatgaatggtgaagatgtgaagaaccaggccaacaccatctgcgc
gcacatggctgagaaagccagcggtacctatcgcctgctgtttgtgccgggtcagctgagccagggtgcttatagcagcatcattgaggaa
ccgagcgtgaaggaagtgctgaacaccatcaaaagcgccagcatgctggtgcacggcattggcgaggcgaagaccatggctcagcgt
cgcaacaccccgctggaagacctgaagaaaatcgacgataacgatgccgtgaccgaggccttcggctactacttcaacgcggacggtg

aagtggtgcacaaggtgcacagcgtgggtatgcagctggacgatatcgacgccattccggatatcattgccgtggcgggcggtagcagc
aaagctgaggccattgaagcgtactttaagaaaccgcgtaataccgtgctggtgaccgatgagggtgctgccaagaaactgctgcgcgat
gaa

18>tctaatgctaaagatgttctgggcctgaccctgctggagaagaccctgaaagaacgtctgaacctgaaagatgctatcattgtgagcg
gcgacagcgatcagagcccgtgggtgaagaaagagatgggtcgtgccgcggtggcgtgcatgaagaaacgcttcagcggcaagaac
atcgtggctgtgaccggcggtaccaccattgaggcggtggctgaaatgatgaccccggacagcaaaaaccgtgagctgctgtttgtgcca
gctcgcggctacaacagcgacaacgtctatatcatggccgacaagcagaagaacggcatcaaggccaacttcaagatccgccacaac
gtcgaggacggcagcgtgcagctcgccgaccactaccagcagaacacccccatcggcgacggccccgtgctgctgcccgacaacca
ctacctgagcttccagtccgtcctgagcaaagaccccaacgagaagcgcgatcacatggtcctgctggagttcgtgaccgccgccgggat
cactctcggcatggacgagctgtacaacgtggatggcggtagcggtggcaccggcagcaagggcgaggagctgttcaccggggtggtg
cccatcctggtcgagctggacggcgacgtaaacggccacaagttcagcgtgtccggcgagggcgagggcgatgccacctacggcaag
ctgaccctgaagctgatctgcaccaccggcaagctgcccgtgccctggcccaccctcgtgaccaccctcggctacggcctgaagtgcttc
gcccgctaccccgaccacatgaagcagcacgacttcttcaagtccgccatgcccgaaggctacgtccaggagcgcaccatcttcttcaag
gacgacggcaactacaagacccgcgccgaggtgaagttcgagggcgacaccctggtgaaccgcatcgagctgaagggcatcggcttc
aaggaggacggcaacatcctggggcacaagctggagtacaacatgaatggtgaagatgtgaagaacgccgccaacaccatctgcgc
gcacatggctgagaaagccagcggtacctatcgcctgctgtttgtgccgggtcagctgagccagggtgcttatagcagcatcattgaggaa
ccgagcgtgaaggaagtgctgaacaccatcaaaagcgccagcatgctggtgcacggcattggcgaggcgaagaccatggctcagcgt
cgcaacaccccgctggaagacctgaagaaaatcgacgataacgatgccgtgaccgaggccttcggctactacttcaacgcggacggtg
aagtggtgcacaaggtgcacagcgtgggtatgcagctggacgatatcgacgccattccggatatcattgccgtggcgggcggtagcagc
aaagctgaggccattgaagcgtactttaagaaaccgcgtaataccgtgctggtgaccgatgagggtgctgccaagaaactgctgcgcgat
gaa

19>tctaatgctaaagatgttctgggcctgaccctgctggagaagaccctgaaagaacgtctgaacctgaaagatgctatcattgtgagcg
gcgacagcgatcagagcccgtgggtgaagaaagagatgggtcgtgccgcggtggcgtgcatgaagaaacgcttcagcggcaagaac
atcgtggctgtgaccggcggtaccaccattgaggcggtggctgaaatgatgaccccggacagcaaaaaccgtgagctgctgtttgtgcca
gctcgcggctacaacagcgacaacgtctatatcatggccgacaagcagaagaacggcatcaaggccaacttcaagatccgccacaac
gtcgaggacggcagcgtgcagctcgccgaccactaccagcagaacacccccatcggcgacggccccgtgctgctgcccgacaacca
ctacctgagcttccagtccgtcctgagcaaagaccccaacgagaagcgcgatcacatggtcctgctggagttcgtgaccgccgccgggat
cactctcggcatggacgagctgtacaacgtggatggcggtagcggtggcaccggcagcaagggcgaggagctgttcaccggggtggtg
cccatcctggtcgagctggacggcgacgtaaacggccacaagttcagcgtgtccggcgagggcgagggcgatgccacctacggcaag
ctgaccctgaagctgatctgcaccaccggcaagctgcccgtgccctggcccaccctcgtgaccaccctcggctacggcctgaagtgcttc
gcccgctaccccgaccacatgaagcagcacgacttcttcaagtccgccatgcccgaaggctacgtccaggagcgcaccatcttcttcaag
gacgacggcaactacaagacccgcgccgaggtgaagttcgagggcgacaccctggtgaaccgcatcgagctgaagggcatcggcttc
aaggaggacggcaacatcctggggcacaagctggagtacaacatgaatggtgaagatgtgaagaacaccgccaacaccatctgcgc
gcacatggctgagaaagccagcggtacctatcgcctgctgtttgtgccgggtcagctgagccagggtgcttatagcagcatcattgaggaa
ccgagcgtgaaggaagtgctgaacaccatcaaaagcgccagcatgctggtgcacggcattggcgaggcgaagaccatggctcagcgt
cgcaacaccccgctggaagacctgaagaaaatcgacgataacgatgccgtgaccgaggccttcggctactacttcaacgcggacggtg
aagtggtgcacaaggtgcacagcgtgggtatgcagctggacgatatcgacgccattccggatatcattgccgtggcgggcggtagcagc
aaagctgaggccattgaagcgtactttaagaaaccgcgtaataccgtgctggtgaccgatgagggtgctgccaagaaactgctgcgcgat
gaa

20>tctaatgctaaagatgttctgggcctgaccctgctggagaagaccctgaaagaacgtctgaacctgaaagatgctatcattgtgagcg
gcgacagcgatcagagcccgtgggtgaagaaagagatgggtcgtgccgcggtggcgtgcatgaagaaacgcttcagcggcaagaac
atcgtggctgtgaccggcggtaccaccattgaggcggtggctgaaatgatgaccccggacagcaaaaaccgtgagctgctgtttgtgcca
gctcgcggctacaacagcgacaacgtctatatcatggccgacaagcagaagaacggcatcaaggccaacttcaagatccgccacaac
gtcgaggacggcagcgtgcagctcgccgaccactaccagcagaacacccccatcggcgacggccccgtgctgctgcccgacaacca
ctacctgagcttccagtccgtcctgagcaaagaccccaacgagaagcgcgatcacatggtcctgctggagttcgtgaccgccgccgggat
cactctcggcatggacgagctgtacaacgtggatggcggtagcggtggcaccggcagcaagggcgaggagctgttcaccggggtggtg
cccatcctggtcgagctggacggcgacgtaaacggccacaagttcagcgtgtccggcgagggcgagggcgatgccacctacggcaag
ctgaccctgaagctgatctgcaccaccggcaagctgcccgtgccctggcccaccctcgtgaccaccctcggctacggcctgaagtgcttc
gcccgctaccccgaccacatgaagcagcacgacttcttcaagtccgccatgcccgaaggctacgtccaggagcgcaccatcttcttcaag
gacgacggcaactacaagacccgcgccgaggtgaagttcgagggcgacaccctggtgaaccgcatcgagctgaagggcatcggcttc
aaggaggacggcaacatcctggggcacaagctggagtacaacatgaatggtgaagatgtgaagaacgatgccaacaccatctgcgc
gcacatggctgagaaagccagcggtacctatcgcctgctgtttgtgccgggtcagctgagccagggtgcttatagcagcatcattgaggaa
ccgagcgtgaaggaagtgctgaacaccatcaaaagcgccagcatgctggtgcacggcattggcgaggcgaagaccatggctcagcgt
cgcaacaccccgctggaagacctgaagaaaatcgacgataacgatgccgtgaccgaggccttcggctactacttcaacgcggacggtg
aagtggtgcacaaggtgcacagcgtgggtatgcagctggacgatatcgacgccattccggatatcattgccgtggcgggcggtagcagc
aaagctgaggccattgaagcgtactttaagaaaccgcgtaataccgtgctggtgaccgatgagggtgctgccaagaaactgctgcgcgat
gaa

21>tctaatgctaaagatgttctgggcctgaccctgctggagaagaccctgaaagaacgtctgaacctgaaagatgctatcattgtgagcg
gcgacagcgatcagagcccgtgggtgaagaaagagatgggtcgtgccgcggtggcgtgcatgaagaaacgcttcagcggcaagaac

atcgtggctgtgaccggcggtaccaccattgaggcggtggctgaaatgatgaccccggacagcaaaaaccgtgagctgctgtttgtgcca
gctcgcggcttgcttggtgaagaaggcgtaaacagcgacaacgtctatatcatggccgacaagcagaagaacggcatcaagaccaact
tcaagatccgccacaacgtcgaggacggcagcgtgcagctcgccgaccactaccagcagaacacccccatcggcgacggccccgtg
ctgctgcccgacaaccactacctgagcttccagtccgtcctgagcaaagaccccaacgagaagcgcgatcacatggtcctgctggagttc
gtgaccgccgccgggatcactctcggcatggacgagctgtacaacgtggatggcggtagcggtggcaccggcagcaagggcgagga
gctgttcaccggggtggtgcccatcctggtcgagctggacggcgacgtaaacggccacaagttcagcgtgtccggcgagggcgagggc
gatgccacctacggcaagctgaccctgaagctgatctgcaccaccggcaagctgcccgtgccctggcccaccctcgtgaccaccctcgg
ctacggcctgaagtgcttcgcccgctaccccgaccacatgaagcagcacgacttcttcaagtccgccatgcccgaaggctacgtccagg
agcgcaccatcttcttcaaggacgacggcaactacaagacccgcgccgaggtgaagttcgagggcgacaccctggtgaaccgcatcg
agctgaagggcatcgacttcaaggaggacggcaacatcctggggcacaagctggagtacaacgtgccggccaacaccatctgcgcgc
acatggctgagaaagccagcggtacctatcgcctgctgtttgtgccgggtcagctgagccagggtgcttatagcagcatcattgaggaacc
gagcgtgaaggaagtgctgaacaccatcaaaagcgccagcatgctggtgcacggcattggcgaggcgaagaccatggctcagcgtcg
caacaccccgctggaagacctgaagaaaatcgacgataacgatgccgtgaccgaggccttcggctactacttcaacgcggacggtgaa
gtggtgcacaaggtgcacagcgtgggtatgcagctggacgatatcgacgccattccggatatcattgccgtggcgggcggtagcagcaa
agctgaggccattgaagcgtactttaagaaaccgcgtaataccgtgctggtgaccgatgagggtgctgccaagaaactgctgcgcgatga
a

22>tctaatgctaaagatgttctgggcctgaccctgctggagaagaccctgaaagaacgtctgaacctgaaagatgctatcattgtgagcg
gcgacagcgatcagagcccgtgggtgaagaaagagatgggtcgtgccgcggtggcgtgcatgaagaaacgcttcagcggcaagaac
atcgtggctgtgaccggcggtaccaccattgaggcggtggctgaaatgatgaccccggacagcaaaaaccgtgagctgctgtttgtgcca
gctcgcggcttgcttggtgaagaaggcatcaacagcgacaacgtctatatcatggccgacaagcagaagaacggcatcaagaccaact
tcaagatccgccacaacgtcgaggacggcagcgtgcagctcgccgaccactaccagcagaacacccccatcggcgacggccccgtg
ctgctgcccgacaaccactacctgagcttccagtccgtcctgagcaaagaccccaacgagaagcgcgatcacatggtcctgctggagttc
gtgaccgccgccgggatcactctcggcatggacgagctgtacaacgtggatggcggtagcggtggcaccggcagcaagggcgagga
gctgttcaccggggtggtgcccatcctggtcgagctggacggcgacgtaaacggccacaagttcagcgtgtccggcgagggcgagggc
gatgccacctacggcaagctgaccctgaagctgatctgcaccaccggcaagctgcccgtgccctggcccaccctcgtgaccaccctcgg
ctacggcctgaagtgcttcgcccgctaccccgaccacatgaagcagcacgacttcttcaagtccgccatgcccgaaggctacgtccagg
agcgcaccatcttcttcaaggacgacggcaactacaagacccgcgccgaggtgaagttcgagggcgacaccctggtgaaccgcatcg
agctgaagggcatcgacttcaaggaggacggcaacatcctggggcacaagctggagtacaacgtgccggccaacaccatctgcgcgc
acatggctgagaaagccagcggtacctatcgcctgctgtttgtgccgggtcagctgagccagggtgcttatagcagcatcattgaggaacc
gagcgtgaaggaagtgctgaacaccatcaaaagcgccagcatgctggtgcacggcattggcgaggcgaagaccatggctcagcgtcg
caacaccccgctggaagacctgaagaaaatcgacgataacgatgccgtgaccgaggccttcggctactacttcaacgcggacggtgaa
gtggtgcacaaggtgcacagcgtgggtatgcagctggacgatatcgacgccattccggatatcattgccgtggcgggcggtagcagcaa
agctgaggccattgaagcgtactttaagaaaccgcgtaataccgtgctggtgaccgatgagggtgctgccaagaaactgctgcgcgatga
a

23>tctaatgctaaagatgttctgggcctgaccctgctggagaagaccctgaaagaacgtctgaacctgaaagatgctatcattgtgagcg
gcgacagcgatcagagcccgtgggtgaagaaagagatgggtcgtgccgcggtggcgtgcatgaagaaacgcttcagcggcaagaac
atcgtggctgtgaccggcggtaccaccattgaggcggtggctgaaatgatgaccccggacagcaaaaaccgtgagctgctgtttgtgcca
gctcgcggcggtcttggtgaagaaggcgtaaacagcgacaacgtctatatcatggccgacaagcagaagaacggcatcaagaccaac
ttcaagatccgccacaacgtcgaggacggcagcgtgcagctcgccgaccactaccagcagaacacccccatcggcgacggccccgtg
ctgctgcccgacaaccactacctgagcttccagtccgtcctgagcaaagaccccaacgagaagcgcgatcacatggtcctgctggagttc
gtgaccgccgccgggatcactctcggcatggacgagctgtacaacgtggatggcggtagcggtggcaccggcagcaagggcgagga
gctgttcaccggggtggtgcccatcctggtcgagctggacggcgacgtaaacggccacaagttcagcgtgtccggcgagggcgagggc
gatgccacctacggcaagctgaccctgaagctgatctgcaccaccggcaagctgcccgtgccctggcccaccctcgtgaccaccctcgg
ctacggcctgaagtgcttcgcccgctaccccgaccacatgaagcagcacgacttcttcaagtccgccatgcccgaaggctacgtccagg
agcgcaccatcttcttcaaggacgacggcaactacaagacccgcgccgaggtgaagttcgagggcgacaccctggtgaaccgcatcg
agctgaagggcatcgacttcaaggaggacggcaacatcctggggcacaagctggagtacaacgtgccggccaacaccatctgcgcgc
acatggctgagaaagccagcggtacctatcgcctgctgtttgtgccgggtcagctgagccagggtgcttatagcagcatcattgaggaacc
gagcgtgaaggaagtgctgaacaccatcaaaagcgccagcatgctggtgcacggcattggcgaggcgaagaccatggctcagcgtcg
caacaccccgctggaagacctgaagaaaatcgacgataacgatgccgtgaccgaggccttcggctactacttcaacgcggacggtgaa
gtggtgcacaaggtgcacagcgtgggtatgcagctggacgatatcgacgccattccggatatcattgccgtggcgggcggtagcagcaa
agctgaggccattgaagcgtactttaagaaaccgcgtaataccgtgctggtgaccgatgagggtgctgccaagaaactgctgcgcgatga
a

## Claims

1. A fructose-1,6-bisphosphate binding protein variant, which:
(1) having a sequence as shown in SEQ ID NO: 1 and having a mutation at one, two, three, four, five or more positions selected from the group consisting of: T148, R175, G176, G177, L178, E180, D181, V182, K183, N184, Q185, F271, and wherein the mutation comprises a modification, substitution or deletion of an amino acid,
(2) is a truncated variant of (1) having amino acids 88 to 340, or
(3) is a sequence having at least 70% sequence identity with the sequence of (1) or (2) and having the mutation described in (1) and retaining the ability to bind to fructose-1,6-bisphosphate, preferably, the mutated positions are selected from 1, 2 or 3, 4, or 5 positions of any one of the following groups: (a) T148, R175, G176, G177, L178, D181, V182, K183, N184, Q185, (b) G177, L178, E180, D181, V182, N184, Q185, F271,
more preferably, the mutation comprises mutations at positions selected from any one of the following groups: (1) G177 and L178, (5) D181 and V182, (6) E180 and D181, (7) N184 and Q185; or, the mutation comprises mutations at positions selected from any one of the following groups: (1) G177, L178, (2) G177, L178 and Q185, (3 )G177, L178 and T148, (4) G177, L178 and F271, (5) D181, V182, (6) E180, D181, (7) N184, Q185, (8) N184, Q185, D181, V182, G177, (9) N184, Q185, G177, (10) N184, Q185, D181, V182,
more preferably, T148 is mutated to S; G177 is mutated to A, I, L, M or N, preferably is mutated to L or M; L178 is mutated to Y, F or N, preferably is mutated to N; E180 is mutated to G; D181 is mutated to F, L or E, preferably is mutated to E; V182 is mutated to F, L or G, preferably is mutated to G; N184 is mutated to V; Q185 is mutated to S, A, T, D or P, preferably is mutated to P; F271 is mutated to Y,
more preferably, the mutation comprises mutations selected from any one of the following groups: (1) G177A and L178Y, (2) G177I and L178F, (3) G177L and L178Y, (4) G177M and L178N, (5) G177N and L178Y, (6) G177M, L178N and Q185S, (7) G177M, L178N and Q185A, (8) G177M, L178N and Q185T, (9) G177M, L178N and Q185D, (10) G177, L178 and T148S, (11) G177, L178 and F271Y, (12) D181F and V182F, (13) D181L and V182L, (14) E180G and D181G, (15) N184V and Q185P, (16) N184V, Q185P, D181E, V182G and G177L, (17) N184V, Q185P and G177L, (18) N184V, Q185P, D181E and V182G.

2. A fructose-1,6-bisphosphate optical probe, comprising a fructose-1,6-bisphosphate sensitive polypeptide and an optically active polypeptide, wherein the optically active polypeptide is located within the sequence of the fructose-1,6-bisphosphate sensitive polypeptide, the fructose-1,6-bisphosphate sensitive polypeptide is a fructose-1,6-bisphosphate binding protein or a functional variant thereof, and the optically active polypeptide is a fluorescent protein or a functional variant thereof; wherein the functional variant of the fructose-1,6-bisphosphate binding protein has a mutation within 7 amino acids from the connection with the optically active polypeptide, and the functional variant of the fluorescent protein has a mutation within 3 amino acids from the connection with the optically active polypeptide.

3. An optical probe according to claim 1, wherein the fructose-1,6-bisphosphate binding protein has the sequence shown in SEQ ID NO: 1 or a truncation having amino acids 88-340 thereof, or a sequence having at least 70% sequence identity therewith and retaining sensitivity to fructose-1,6-bisphosphate.

4. The optical probe according to claim 1, wherein the optically active polypeptide is located between residues 174-185 and/or 201-208 of the fructose-1,6-bisphosphate sensitive polypeptide,
preferably, the optically active polypeptide is located at any one or more of the following positions of the fructose-1,6-bisphosphate sensitive polypeptide: 174/175, 174/176, 174/177, 174/178, 174/179, 174/180, 174/181, 174/182, 174/183, 174/184, 174/185, 175/176, 175/177, 175/178, 175/179, 175/180, 175/181, 175/182, 175/183, 175/184, 175/185 75/185, 176/177, 176/178, 176/179, 176/180, 176/181, 176/182, 176/183, 176/184, 176/185, 177/178, 177/179, 177/180, 177/181, 177/182, 177/183, 177/184, 177/185, 178/179, 178/180, 178/181, 178/182, 178/183, 178/184, 178/185, 179/ 180, 179/181, 179/182, 179/183, 179/184, 179/185, 180/181, 180/182, 180/183, 180/184, 180/185, 181/182, 181/182, 181/183, 181/184, 181/185, 182/183, 182/184, 182/185, 183/184, 183/185, 184/185, 201/202, 201/203, 201/204, 201/205 , 201/206, 201/207, 201/208, 202/203, 202/204, 202/205, 202/206, 202/207, 202/208, 203/204, 203/205, 203/206, 203/207, 203/208, 204/205, 204/206, 204/207, 204/208, 205/206, 205/206, 205/207, 205/208, 206/207, 206/208 and/or 207/208, more preferably, the optically active polypeptide is located at any one or more of the following positions of the fructose-1,6-bisphosphate-sensitive polypeptide: 176/177, 176/178, 177/181, 179/180, 182/184, 182/185, 203/206, 203/207 and 203/208,
preferably, the functional variant of fructose-1,6-bisphosphate binding protein is as described in claim 1;
preferably, the fluorescent protein is selected from yellow fluorescent protein, orange fluorescent protein, red fluorescent protein, green fluorescent protein, blue fluorescent protein, apple red fluorescent protein, and the functional variant of the fluorescent protein has a mutation at the amino acid 1-3; more preferably, the fluorescent protein has a sequence shown in any one of SEQ ID NOs: 2-9, and the functional variant of the fluorescent protein has a mutation at the first amino acid,
preferably, the functional variant of the fluorescent protein comprises a mutation in which the first amino acid of the fluorescent protein is mutated to I or V,
preferably, the fructose-1,6-bisphosphate sensitive polypeptide is a truncated variant of SEQ ID NO: 1 having amino acids 88-340, and the optically active polypeptide is located at any one or more of the following positions of the fructose-1,6-bisphosphate sensitive polypeptide: 176/177, 176/178, 177/181, 179/180, 182/184, 182/185, 203/206, 203/207 and 203/208; more preferably, the optical probe has a sequence shown in any one of SEQ ID NO: 10-18,
preferably, the fructose-1,6-bisphosphate sensitive polypeptide is a truncated variant of SEQ ID NO: 1 having amino acids 88-340, the optically active polypeptide is located at any one or more of the following positions of the fructose-1,6-bisphosphate sensitive polypeptide: 176/177, 177/181, 179/180 or 182/184, and the optical probe has one or more mutations selected from the group consisting of: T148S, G177A, G1771, G177L, G177 M, G177N, L178Y, L178F, L178Y, L178N, L178Y, L178N, E180G, D181E, D181F, D181L, D181G, V182F, V182L, V182G, N184V, Q185A, Q185S, Q185A, Q185T, Q185D, Q185P, F271Y, and the first amino acid of the optically active polypeptide is mutated to V or I,
preferably, the optical probe has a mutation selected from any one of the following groups: (1) G177A and L178Y of the fructose-1,6-bisphosphate sensitive polypeptide, (2) G177I and L178F of the fructose-1,6-bisphosphate sensitive polypeptide, (3) G177L and L178Y of the fructose-1,6-bisphosphate sensitive polypeptide, (4) G177M and L178N of the fructose-1,6-bisphosphate sensitive polypeptide, (5) G177N and L178Y of the fructose-1,6-bisphosphate sensitive polypeptide, (6) G177M, L178N and Q185S of the fructose-1,6-bisphosphate sensitive polypeptide, (7) G177M, L178N and Q185A of the fructose-1,6-bisphosphate sensitive polypeptide, (8) G177M, L178N and Q185T of the fructose-1,6-bisphosphate sensitive polypeptide, (9) G177M, L178N and Q185D of the fructose-1,6-bisphosphate sensitive polypeptide, (10) G177M, L178N and T148S of the fructose-1,6-bisphosphate sensitive polypeptide, (11) G177M, L178N and F271Y of the fructose-1,6-bisphosphate sensitive polypeptide, (12) D181F and V182F of the fructose-1,6-bisphosphate sensitive polypeptide, (13) D181L and V182L of the fructose-1,6-bisphosphate sensitive polypeptide, (14) E180G and D181G of the fructose-1,6-bisphosphate sensitive polypeptide, (15) N184V and Q185P of the fructose-1,6-bisphosphate sensitive polypeptide, (16) N184V, Q185P, D181E, V182G, G177L of the fructose-1,6-bisphosphate sensitive polypeptide, and the first amino acid of the optically active polypeptide is mutated to V, (17) N184V, Q185P, D181E,V182G,G177L of the fructose-1,6-bisphosphate sensitive polypeptide and the first amino acid of the optically active polypeptide is mutated to I, (18) N184V, Q185P and G177L of the fructose-1,6-bisphosphate sensitive polypeptide, and the first amino acid of the optically active polypeptide is mutated to V, (19) N184V, Q185P and G177L of the fructose-1,6-bisphosphate sensitive polypeptide, and the first amino acid of the optically active polypeptide is mutated to I, (20) N184V, Q185P, D181E and V182G of the fructose-1,6-bisphosphate sensitive polypeptide, and the first amino acid of the optically active polypeptide is mutated to V.

5. A nucleic acid molecule comprising:
(a) a coding sequence of the optical probe described in any one of claims 2 to 4,
(b) a complement sequence of (a).

6. A nucleic acid construct comprising the nucleic acid molecule according to claim 5, preferably, the nucleic acid construct is a cloning vector, an expression vector or a recombinant vector.

7. A host cell, wherein the host cell:
(1) expresses the optical probe according to any one of claims 2 to 4;
(2) comprises nucleic acid molecule according to claim 5; and/or
(3) comprises the nucleic acid construct according to claim 6.

8. A detection kit comprising:
(1) the optical probe according to any one of claims 2 to 4;
(2) the nucleic acid sequence according to claim 5;
(3) the nucleic acid construct according to claim 6; or
(4) the host cell according to claim 7;
the detection kit optionally further comprises other reagents required for detecting fructose-1,6-bisphosphate using an optical probe,
preferably, the detection kit further comprises one or more reagents selected from the group consisting of: a buffer, a culture medium, and a fructose-1,6-bisphosphate standard.

9. A method for preparing the optical probe according to any one of claims 2 to 4, comprising: providing the host cell according to claim 7, culturing the host cell under conditions where the optical probe is expressed, and isolating the optical probe.

10. Use of the optical probe according to any one of claims 2 to 4, the nucleic acid sequence described in claim 5, the nucleic acid construct according to claim 6 or the host cell according to claim 7 in detecting fructose-1,6-bisphosphate in a sample, screening compounds, and intracellular/extracellularly localizing fructose-1,6-bisphosphate,
preferably,
the detection of fructose-1,6-bisphosphate in a sample comprises the steps of: contacting the optical probe or host cell with the sample, determining an optical change of the optically active polypeptide, and detecting fructose-1,6-bisphosphate in the sample according to the optical change of the optically active polypeptide,
the screening of compounds comprises the steps of: contacting the optical probe or the host cell with a candidate compound in a system containing fructose-1,6-bisphosphate, determining an optical change of an optically active polypeptide, and screening the compound according to the optical change of the optically active polypeptide; preferably, the screening compound comprises the steps of: contacting the host cell with a candidate compound in a system containing fructose-1,6-bisphosphate, and the optical change of the optically active polypeptide indicates whether the candidate compound regulates the cell's uptake of fructose-1,6-bisphosphate,
the intracellular/extracellular localization of fructose-1,6-bisphosphate comprises the steps of: contacting a system containing fructose-1,6-bisphosphate with the optical probe or the host cell, and detecting the optical change of the optically active polypeptide,
more preferably, the system is a solution system, a cell system or a subcellular system.
